(19) Europäisches Patentamt
European Patent Office
Office européen des brevets

(11) EP 1 415 992 B1

(12) **EUROPEAN PATENT SPECIFICATION**

(45) Date of publication and mention of the grant of the patent:
**02.05.2012 Bulletin 2012/18**

(21) Application number: **02762760.3**

(22) Date of filing: **08.08.2002**

(51) Int Cl.:
*C07D 401/12* (2006.01) *C07D 417/12* (2006.01)
*C07D 417/14* (2006.01) *C07D 401/14* (2006.01)
*C07D 209/42* (2006.01) *C07D 487/04* (2006.01)
*C07D 513/04* (2006.01) *C07D 498/04* (2006.01)
*C07D 513/14* (2006.01) *C07K 5/06* (2006.01)
*C07K 5/08* (2006.01) *A61K 31/428* (2006.01)
*A61K 31/429* (2006.01) *A61K 31/4365* (2006.01)
*A61K 31/437* (2006.01) *A61K 31/444* (2006.01)
*A61K 31/4545* (2006.01) *A61K 31/454* (2006.01)
*A61K 31/4439* (2006.01) *A61P 7/02* (2006.01)

(86) International application number:
**PCT/JP2002/008119**

(87) International publication number:
**WO 2003/016302 (27.02.2003 Gazette 2003/09)**

(54) **DIAMINE DERIVATIVES**

DIAMINDERIVATE

DERIVES DE DIAMINE

(84) Designated Contracting States:
**AT BE BG CH CY CZ DE DK EE ES FI FR GB GR IE IT LI LU MC NL PT SE SK TR**

(30) Priority: **09.08.2001 JP 2001243046**
**09.10.2001 JP 2001311808**
**28.12.2001 JP 2001398708**
**20.03.2002 PCT/JP02/02683**
**20.06.2002 PCT/JP02/06141**

(43) Date of publication of application:
**06.05.2004 Bulletin 2004/19**

(73) Proprietor: **Daiichi Sankyo Company, Limited**
**Chuo-ku**
**Tokyo (JP)**

(72) Inventors:
- **OHTA, Toshiharu,**
**DAIICHI PHARMACEUT. CO. LTD**
**Tokyo 134-8630 (JP)**
- **KOMORIYA, Satoshi,**
**DAIICHI PHARMACEUT. CO. LTD**
**Tokyo 134-8630 (JP)**
- **YOSHINO, Toshiharu,**
**DAIICHI PHARMACEUT. CO. LTD**
**Tokyo 134-8630 (JP)**
- **UOTO, Kouichi,**
**DAIICHI PHARMACEUT. CO. LTD**
**Tokyo 134-8630 (JP)**
- **NAKAMOTO, Yumi,**
**DAIICHI PHARMACEUT. CO. LTD**
**Tokyo 134-8630 (JP)**
- **NAITO, Hiroyuki,**
**DAIICHI PHARMACEUT. CO. LTD**
**Tokyo 134-8630 (JP)**
- **MOCHIZUKI, Akiyoshi,**
**DAIICHI PHARMACEUT. CO.LTD**
**Tokyo 134-8630 (JP)**
- **NAGATA, Tsutomu,**
**DAIICHI PHARMACEUT. CO. LTD**
**Tokyo 134-8630 (JP)**
- **KANNO, Hideyuki,**
**DAIICHI PHARMACEUT. CO. LTD**
**Tokyo 134-8630 (JP)**
- **HAGINOYA, Noriyasu,**
**DAIICHI PHARMACEUT. CO. LTD**
**Tokyo 134-8630 (JP)**
- **YOSHIKAWA, Kenji,**
**DAIICHI PHARMACEUT. CO. LTD**
**Tokyo 134-8630 (JP)**
- **NAGAMOCHI, Masatoshi,**
**DAIICHI PHARMACEUT. CO. LTD**
**Tokyo 134-8630 (JP)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 415 992 B1

- KOBAYASHI, Syozo,
  DAIICHI PHARMACEUT. CO. LTD
  Tokyo 134-8630 (JP)
- ONO, Makoto,
  DAIICHI PHARMACEUT. CO. LTD
  Tokyo 134-8630 (JP)

(74) Representative: **Hartz, Nikolai**
**Wächtershäuser & Hartz**
**Patentanwaltspartnerschaft**
**Ottostrasse 4**
**80333 München (DE)**



(56) References cited:

**EP-A- 1 405 852** **EP-A1- 0 602 306**
**EP-A1- 0 947 510** **WO-A-00/00498**
**WO-A-99/51614** **WO-A-03/000657**
**WO-A-03/026652** **WO-A-03/048081**
**WO-A-03/048158** **WO-A-03/099276**
**WO-A1-00/09480** **WO-A1-00/59913**
**WO-A1-00/76942** **WO-A1-01/07440**
**WO-A1-01/74774** **WO-A1-92/04017**
**WO-A1-98/45262** **WO-A1-98/57952**
**WO-A1-99/54308** **WO-A2-00/64902**
**WO-A2-86/07257** **WO-A2-94/20062**
**WO-A2-97/10853** **WO-A2-99/32225**
**US-A- 6 054 065**

- **PANDEY B R ET AL: "Interrelationship between anticonvulsant and enzyme inhibitor properties of N-methyl-N-[2-(1-arylthiocarbamido) cyclopenty]nitrobenzamides" PHARMACOLOGICAL RESEARCH COMMUNICATIONS, ITALIAN PHARMACOLOGICAL SOCIETY, IT, vol. 13, no. 1, 1981, pages 65-74, XP002977669 ISSN: 0031-6989**
- **BIED, C. ET AL: "Chiral amino-urea derivatives of (1R,2R)-1,2-diaminocyclohexane as ligands in the ruthenium catalyzed asymmetric reduction of aromatic ketones by hydride transfer" TETRAHEDRON: ASYMMETRY , 12(2), 329-336 CODEN: TASYE3; ISSN: 0957-4166, 2001, XP004230915**
- **SMITH, PAUL J. ET AL: "Substrate-specific catalysis via ion pairs" ANGEWANDTE CHEMIE INT. ED. ENGL., 1993, 32(11), 1648-50, 1993, XP002384367**
- **GASPARRINI FRANCESCO ET AL.: 'Enantioselective chromatography on brush-type chiral stationary phases containing totally synthetic selectors theoretical aspects and practical applications' JOURNAL OF CHROMATOGRAPHY A vol. 724, 1996, pages 79 - 90, XP004039547**
- **TORNEIRO MERCEDES AND STILL CLARK W.: 'Simple synthetic receptors that bind peptides in water' TETRAHEDRON vol. 53, no. 26, 1997, pages 8739 - 8750, XP004106087**

## Description

TECHNICAL FIELD

The present disclosure relates to novel compounds which inhibit activated blood coagulation factor X (hereinafter abbreviated as "FXa") to exhibit a potent anticoagulant effect and can be orally administered, and anticoagulants or agents for preventing and/or treating thrombosis or embolism, which comprise such a novel compound as an active ingredient.

BACKGROUND ART

WO 00/09480 discloses sulfonyl derivatives having FXa - inhibitory effects, which are useful as anticoagulant agents.

In unstable angina, cerebral infarction, cerebral embolism, myocardial infarction, pulmonary infarction, pulmonary embolism, Buerger's disease, deep venous thrombosis, disseminated intravascular coagulation syndrome, thrombus formation after valve replacement, reocclusion after angioplasty and thrombus formation during extracorporeal circulation, hypercoagulable state is a pivotal factor. Therefore, there is a demand for development of excellent anticoagulants which have good dose responsiveness, long duration, low risk of hemorrhage. and little side effects and fast onset of sufficient effects even by oral administration (Thrombosis Research, Vol. 68, pp. 507-512, 1992).

Based on the research of anticoagulants worked through various mechanism of action, it is suggested that FXa inhibitors are promising anticoagulants. A blood coagulation system comprises a series of reactions that a great amount of thrombin is produced through an amplification process by multi-stage enzyme reactions to form insoluble fibrin. In an endogenous system, activated factor IX activates into factor X on a phospholipid membrane in the presence of activated factor VIII and calcium ions after multi-stage reactions subsequent to activation of a contact factor. In an exogenous system, activated factor VII activates factor X in the presence of a tissue factor. More specifically, the activation of the factor X into FXa in the coagulation system is a crucial reaction in the formation of thrombin. The activated factor X (FXa) limitedly decomposes prothrombin to produce thrombin in the both systems. Since the produced thrombin activates coagulation factors in the upper stream, the formation of thrombin is more amplified. As described above, since the coagulation system in the upper stream of FXa is divided into the endogenous system and the exogenous system, production of FXa cannot be sufficiently inhibited by inhibiting enzymes in the coagulation system in the upper stream of FXa, leading to production of thrombin. Since the coagulation system comprises self-amplification reactions, inhibition of the coagulation system can be more efficiently achieved by inhibiting FXa in the upper stream of thrombin than the inhibition of thrombin (Thrombosis Research, Vol. 15, pp. 617-629, 1979).

An another excellent point of FXa inhibitors is a great difference between an effective dose in a thrombosis model and a dose elongating bleeding time in an experimental hemorrhagic model. From this experimental result, FXa inhibitors are considered to be anticoagulants having low risk of hemorrhage.

Various compounds have been reported as FXa inhibitors. It is known that antithrombin III and antithrombin III dependent pentasacchrides can generally not inhibit prothrombinase complexes which play a practical role in the thrombus formation in a living body (Thrombosis Research, Vol. 68, pp. 507-512, 1992; Journal of Clinical Investigation, Vol. 71, pp. 1383-1389, 1983; Mebio, Vol. 14, the August number, pp. 92-97). In addition, they do not exhibit effectiveness by oral administration. Tick anticoagulant peptide (TAP) (Science, Vol. 248, pp. 593-596, 1990) and antistasin (AST) (Journal of Biological Chemistry, Vol. 263, pp. 10162-10167, 1988) isolated from mites or leeches, which are bloodsuckers, also inhibit Fxa and exhibit anti-thrombotic effects against venous thrombosis and arterial thrombosis. However, these compounds are high-molecular weight peptides and unavailable in oral administration. As described above, development of antithrombin III independent low-molecular weight FXa inhibitors which directly inhibit coagulation factors has been conducted.

It is therefore an object of the present invention to provide a novel compound which has a potent FXa-inhibiting effect and exhibits an anti-thrombotic effect quickly, sufficiently and persistently by oral administration.

DISCLOSURE OF THE INVENTION

The present inventors have investigated synthesis and pharmacological effects of novel FXa inhibitors. As a result, diamine derivatives, salts thereof, and solvates and N-oxides thereof, which exhibit potent FXa-inhibiting effect and anticoagulant effect, have been found. It has also been found that these compounds promptly, persistently and potently inhibit FXa and exhibit potent anticoagulant effect and anti-thrombotic effect by oral administration, and are hence useful as prophylactics and remedies for various diseases based on thromboembolism, thus leading to completion of the present invention.

This invention considers a compound represented by the general formula (1):

$$Q^1\text{-}Q^2\text{-}T^0\text{-}N(R^1)\text{-}Q^3\text{-}N(R^2)\text{-}T^1\text{-}Q^4 \quad (1)$$

wherein
$R^1$ and $R^2$ represent a hydrogen atom;
$Q^1$ represents a specific unsaturated, bicyclic fused heterocyclic group which is substituted;
$Q^2$ represents a single bond;
$Q^3$ represents the following group:

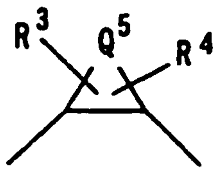

in which $Q^5$ means an alkylene group having 4 carbon atoms; $R^3$ is a specific carboxyl group; and $R^4$ is a hydrogen atom;
$Q^4$ represents a specific heteroaryl group which is substituted;
$T^0$ represents a carbonyl group; and
$T^1$ represents a group -C(=O)-C(=O)-N(R')-, in which R' means a hydrogen atom;
a salt thereof, a solvate thereof, or an N-oxide thereof.

Namely, the present invention provides a compound having a stereochemical structural formula, including a salt thereof, which is:

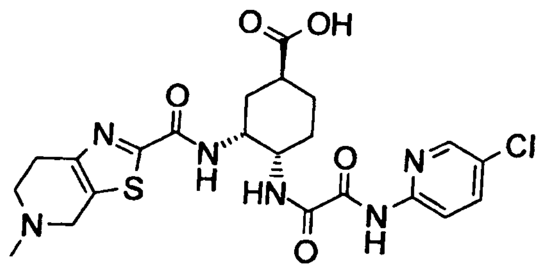

This invention also considers a medicine, an activated blood coagulation factor X inhibitor, an anticoagulant, an agent for preventing and/or treating thrombosis or embolism and an agent for preventing and/or treating cerebral infarction, cerebral embolism, myocardial infarction, angina pectoris, pulmonary infarction, pulmonary embolism, Buerger's disease, deep venous thrombosis, disseminated intravascular coagulation syndrome, thrombus formation after valve or joint replacement, thrombus formation and reocclusion after angioplasty, systemic inflammatory response syndrome (SIRS), multiple organ dysfunction syndrome (MODS), thrombus formation during extracorporeal circulation, or blood clotting upon blood gathering, which each comprises the compound represented by the general formula (1), the salt thereof, or the solvate thereof.

This invention further provides a composition comprising a compound represented by the general formula (1) and a pharmaceutically acceptable carrier.

This invention still further considers the use of the compound represented by the general formula (1), the salt thereof, or the solvate thereof for preparation of a medicine.

A method for treating thrombosis or embolism, which comprises administering an effective amount of the compound represented by the general formula (1), the salt thereof, or the solvate thereof is also disclosed.

BEST MODE FOR CARRYING OUT THE INVENTION

No particular limitation is imposed on salts of the compound of the present invention represented by the general formula (1) so far as they are pharmaceutically acceptable salts. However, specific examples thereof include mineral acid salts such as hydrochlorides, hydrobromides, hydriodides, phosphates, nitrates and sulfates; benzoates; organic sulfonates such as methanesulfonates, 2-hydroxyethanesulfonates and p-toluenesulfonates; and organic carboxylates

such as acetates, propanoates, oxalates, malonates, succinates, glutarates, adipates, tartrates, maleates, malates and mandelates. The compound represented by the general formula (1) has an acidic group and may be a salt of alkali metal ions or alkaline earth metal ions. No particular limitation is imposed on the solvates thereof so far as they are pharmaceutically acceptable solvates. As specific examples thereof, however, may be mentioned hydrates and solvates with ethanol.

The preparation process of the diamine derivatives (1) will hereinafter be described.

[Preparation Process 1] (REFERENCE)

A compound represented by the general formula (1), a salt thereof, a solvate thereof, or an N-oxide thereof can be prepared in accordance with, for example, the following process:

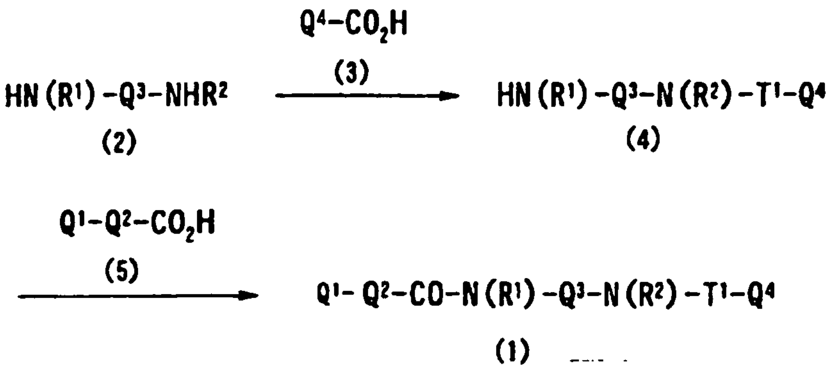

wherein $Q^1$, $Q^2$, $Q^3$, $Q^4$ , $R^1$ and $R^2$ have the same meanings as defined above, and $T^1$ represents a carbonyl group.

A mixed acid anhydride, acid halide, activated ester or the like, which is derived from carboxylic acid (3), may react with diamine (2), giving compound (4). The resultant compound (4) may react with carboxylic acid (5) under the same conditions, giving compound (1) according to the present invention. In the above reaction steps, reagents and conditions, which are generally used in peptide synthesis, may be applied. The mixed acid anhydride can be prepared by, for example, reaction of a chloroformate such as ethyl chloroformate or isobutyl chloroformate with carboxylic acid (3) in the presence of a base. The acid halide can be prepared by treating carboxylic acid (3) with an acid halide such as thionyl chloride or oxalyl chloride. The activated ester includes various kinds of esters. Such an ester can be prepared by, for example, reaction of a phenol such as p-nitrophenol, N-hydroxybenzotriazol, or N-hydroxysccinimide with carboxylic acid (3) using a condensing agent such as N,N'-dicyclohexylcarbodiimide or 1-ethyl-3-(3-dimethylaminopropyl) carbodiimide hydrochloride. The activated ester can also be prepared by reaction of carboxylic acid (3) with pentafluorophenyl trifluoroacetate or the like, reaction of carboxylic acid (3) with 1-benzotriazolyloxytripyrrolidinophosphonium hexafluorophosphite, reaction of carboxylic acid (3) with diethyl cyanophosphonate (Shioiri method), reaction of carboxylic acid (3) with triphenylphosphine and 2,2'-dipyridyl disulfide (Mukaiyama method) or the like. The thus-obtained mixed acid anhydride, acid halide or activated ester of carboxylic acid (3) may react with diamine (2) at -78°C to 150°C in the presence of a proper base in an inert solvent, giving compound (4). Thus-obtained compound (4) may react with a mixed acid anhydride, acid halide or activated ester of carboxylic acid (5) under the same conditions, giving compound (1). The reagents and reaction conditions in the reaction of compound (4) with carboxylic acid (5) are the same as those in the reaction of diamine (2) with carboxylic acid (3).

As specific examples of the base used in each of the above mentioned step, may be carbonates of alkali metals or alkaline earth metals, such as sodium carbonate and potassium carbonate, alkali metal alkoxides such as sodium ethoxide and potassium butoxide, alkali metal hydroxides such as sodium hydroxide and potassium hydroxide, and hydrides of alkali metals or alkaline earth metals, such as sodium hydride and potassium hydride; organic metal bases exemplified by alkyllithium such as n-butyllithium, and dialkylaminolithium such as lithium diisopropylamide; organic metal bases exemplified by bis(silyl)amine, such as lithiumbis(trimethylsilyl)amide; and organic bases such as pyridine, 2,6-lutidine, collidine, 4-dimethylaminopyridine, triethylamine, N-methylmorpholine, diisopropylethylamine and diazabicyclo[5.4.0]undec-7-ene (DBU).

Examples of the inert solvent used in this reaction include alkyl halide type solvents such as dichloromethane, chloroform and carbon tetrachloride, etheric solvents such as tetrahydrofuran, 1,2-dimethoxyethane and dioxane, aromatic solvents such as benzene and toluene, and amide solvents such as N,N-dimethylformamide, N,N-dimethylacetamide and N-methylpyrrolidin-2-one. In addition to these solvent, a sulfoxide solvent such as dimethyl sulfoxide or sulfolane,

a ketone solvent such as acetone or methyl ethyl ketone, or the like may be used in some cases.

[Preparation Process 2] (REFERENCE)

Compound (1) can also be prepared in accordance with the following process:

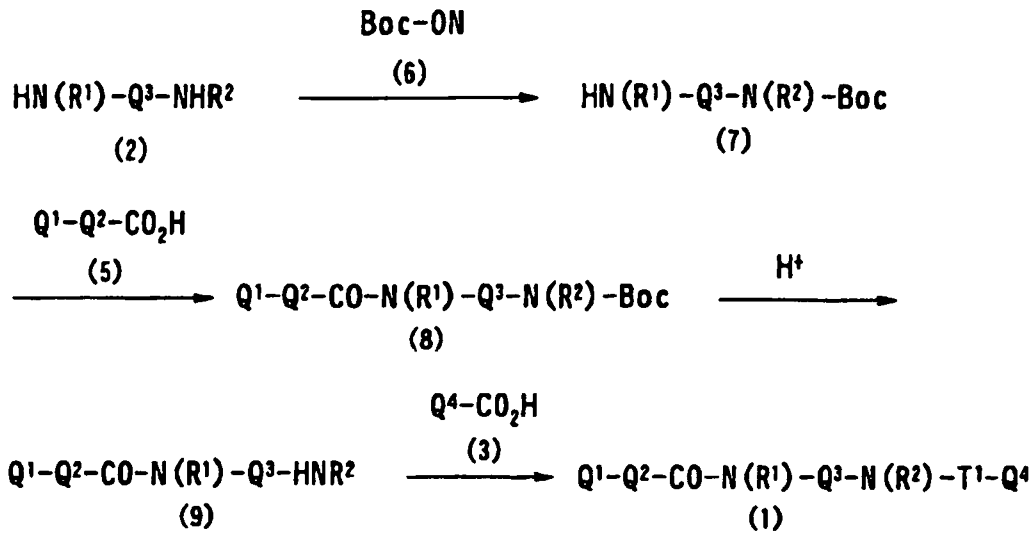


(2) (7) (8) (9) (1)

wherein $Q^1$, $Q^2$, $Q^3$, $Q^4$, $R^1$ and $R^2$ have the same meanings as defined above, $T^1$ represents a carbonyl group, Boc represents a tert-butoxycarbonyl group, and Boc-ON represents a 2-(tert-butoxycarbonyloxyimino)-2-phenylacetonitrile.

As described above, diamine (2) is treated with Boc-ON (6) to prepare compound (7) in which one of 2 amino groups has been protected with tert-butoxycarbonyl group. The resultant compound (7) reacts with carboxylic acid (5) and affords compound (8). Compound (8) is successively treated with an acid to give compound (9). Compound (9) then reacts with the carboxylic acid (3), giving compound (1). Compound (7) can be prepared by a reaction at -10°C to 40°C in the presence of triethylamine in a solvent such as dichloromethane. Reaction of compound (7) with the mixed acid anhydride, acid halide or activated ester of the carboxylic acid (5) is carried out using the same reagents and reaction conditions as those described in Preparation Process 1, whereby compound (8) can be prepared. The resultant compound (8) is treated with trifluoroacetic acid or the like at -20°C to 70°C, whereby amine (9) can be prepared. In the reaction of the resultant amine (9) with carboxylic acid (3), the same reagents and conditions as those described in Preparation Process 1 may be used.

By the way, the tert-butoxycarbonyl group of compound (7) may be replaced by other amino-protecting groups. In this case, reagent (6) is also changed to other reagents, and reaction conditions and the like according to the reagents must be used. As examples of other protecting groups for amino groups, may be mentioned alkanoyl groups such as an acetyl group, alkoxycarbonyl groups such as methoxycarbonyl and ethoxycarbonyl groups, arylmethoxycarbonyl groups such as benzyloxycarbonyl, p-methoxybenzyloxycarbonyl and p- or o-nitrobenzyloxy-carbonyl groups, arylmethyl groups such as benzyl and triphenylmethyl groups, aroyl groups such as a benzoyl group, and arylsulfonyl groups such as 2,4-dinitro-benzenesulfonyl and o-nitrobenzenesulfonyl groups. These protecting groups may be chosen for use according to the nature and the like of the compound of which amino group is to be protected. Upon leaving such a protecting group, reagents and conditions may be employed according to the protecting group.

[Preparation Process 3]

In the compounds (1) geometrical isomers of trans-form and cis-form in the relation between position 1 and position 2 are present when $Q^3$ is the following group:

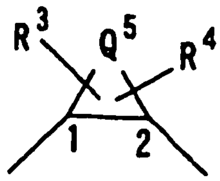

wherein $R^3$, $R^4$ and $Q^5$ have the same meanings as defined above, and numerals 1 and 2 indicate positions.

The preparation processes of such compounds (1) having the trans-form and the cis-form will hereinafter be described.

<Preparation process of trans-form>

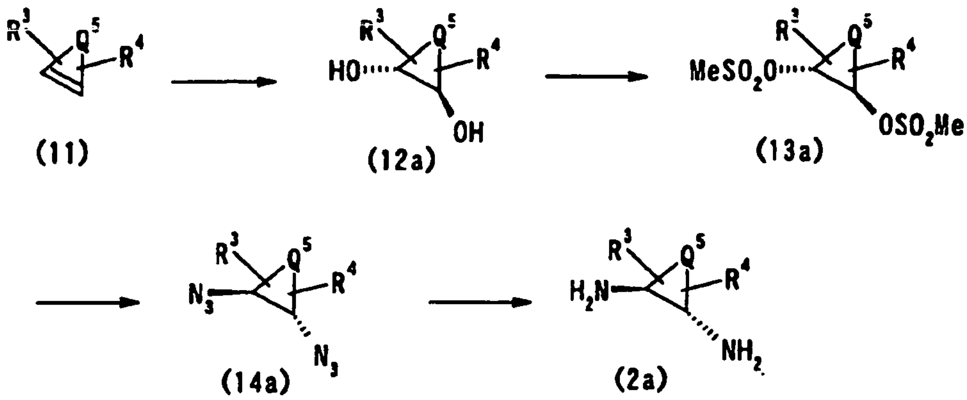

wherein $Q^5$, $R^3$ and $R^4$ have the same meanings as defined above.

As an example of preparation of trans-diol (12a) from cyclic alkene (11), conversion from, for example, cyclohexene to trans-cyclohexanediol (Organic Synthesis, 1995, Vol. III, p. 217) is known. As an example of preparation of trans-diamine (2a) from trans-diol (12a), conversion from trans-cyclopentanediol to trans-cyclopentanediamine (WO98/30574) is reported. Trans-diamine (2a) can be prepared from te cyclic alkene (11) according to these reports.

Trans-diamine (2a) prepared in accordance with the above-described process can be converted into trans-compound (1) by any of the above-described Preparation Processes 1 or 2.

<Preparation process of cis-form>

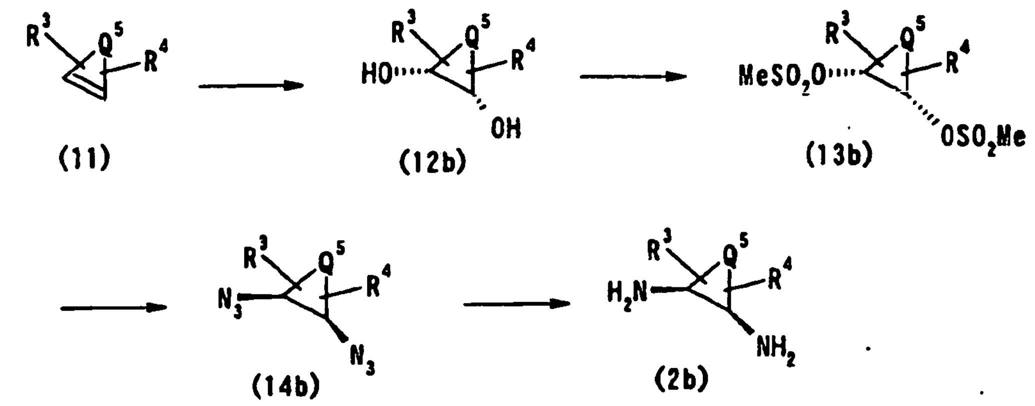

(11) (12b) (13b) (14b) (2b)

wherein $Q^5$, $R^3$ and $R^4$ have the same meanings as defined above, and numerals.

As an example of preparation of cis-diol (12b) from cyclic alkene (11), conversion from cyclohexene to cis-cyclohexanediol (J. Org. Chem., 1998, Vol. 63, p. 6094) and the like is known. As an example of preparation of cis-diamine (2b) from cis-diol (12a), conversion from cis-cyclopentanediol to cis-cyclopentanediamine (WO98/30574) and the like is reported. Cis-diamine (2b) can be prepared from cyclic alkene (11) according to these reports.

Cis-diamine (2b) prepared in accordance with the above-described process can be converted into the cis-compound (1) by any of the above-described Preparation

Processes 1 or 2.

[Preparation Process 4]

As described above, either cis-form or trans-form generated in $Q^3$ may be present in the compounds (1), and so geometrical isomers are present. Further, optical isomers may be present in the respective geometrical isomers. The preparation process of an optically active substance will hereinafter be described.

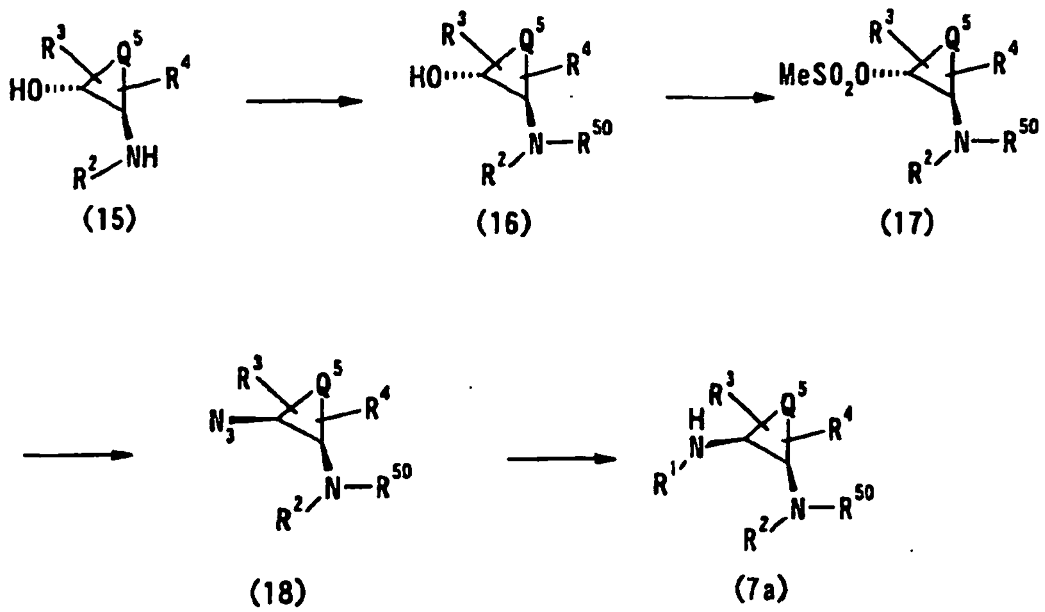

(15) (16) (17) (18) (7a)

wherein $Q^5$, $R^1$, $R^2$, $R^3$ and $R^4$ have the same meanings as defined above, and $R^{50}$ represents a protecting group for amino group.

With respect to the preparation process of optically active aminoalcohol derivative (15) of 1,2-trans-form, for example, the preparation process of optically active 1,2-trans-2-aminocyclopentanol from cyclopentene oxide or the preparation process of optically active 1,2-trans-2-aminocyclohexanol from cyclohexene oxide is known (Tetrahedron: Asymmetry, 1996, Vol. 7, p. 843; J. Org. Chem., 1985, Vol. 50, p. 4154; J. Med. Chem., 1998, Vol. 41, p. 38). When the amino group of optically active aminoalcohol derivative (15) prepared by such an already known process or by applying such a process reacts with a proper protecting reagent, compound (16) can be produced. As a protecting group corresponding to $R^{50}$ in compound (16), is preferred, among the ordinary acyl type protecting groups, an alkoxycarbonyl group such as methoxycarbonyl, ethoxycarbonyl, tert-butoxycarbonyl group and the like, an arylmethoxycarbonyl group such as benzyloxycarbonyl, p-methoxybenzyloxycarbonyl, p- or o-nitrobenzyloxy-carbonyl group and the like, or an arylsulfonyl group such as 2,4-dinitrobenzenesulfonyl, o-nitrobenzenesulfonyl group and the like. When the amino group is protected with, for example, a tert-butoxycarbonyl group, aminoalcohol derivative (15) may react with di-tert-butyl dicarbonate at -78°C to 50°C in an inert solvent, giving compound (16). The inert solvent may be suitably chosen for use from those described in Preparation Process 1.

Compound (16) may react with methanesulfonyl chloride at -78°C to 50°C in the presence of a base in an inert solvent, giving compound (17). The inert solvent may be suitably chosen for use from those described in Preparation Process 1. As the base, is preferred an organic base such as pyridine, 2,6-lutidine, collidine, 4-dimethylaminopyridine, triethylamine, N-methylmorpholine, diisopropylethylamine and diazabicyclo[5.4.0]undec-7-ene (DBU) and the like.

Compound (17) may react with sodium azide at -10°C to 150°C in a proper solvent, giving compound (18). As the solvent, an amide solvent such as N,N-dimethylformamide, N,N-dimethylacetamide or N-methylpyrrolidin-2-one, an alcoholic solvent such as methanol or ethanol, an etheric solvent such as tetrahydrofuran, 1,2-dimethoxyethane or dioxane, benzenoid solvent such as toluene, a carbon halogenide such as dichloromethane, chloroform or carbon tetrachloride, acetone, dimethyl sulfoxide, or a mixed solvent of such a solvent with water is suitable.

As a process for converting azide derivative (18) into compound (7a), there are many processes such as a process of conducting hydrogenation with a palladium catalyst, Raney nickel catalyst or platinum catalyst, a reaction using a reducing agent such as lithium aluminum hydride, sodium borohydride or zinc borohydride, a reaction using zinc in the presence of nickel chloride or cobalt chloride, a reaction using triphenylphosphine and the like. Suitable reaction conditions may be selected according to the nature of the compound. For example, azide derivative (18) is hydrogenated at a temperature of -10°C to 70°C using 1 to 20% palladium carbon as a catalyst in a proper solvent, whereby compound (7a) can be prepared. The hydrogen pressure may be raised higher than atmospheric pressure. As the solvent, an alcoholic solvent such as methanol or ethanol, an etheric solvent such as tetrahydrofuran, 1,2-dimethoxyethane or dioxane, an amide solvent such as N,N-dimethylformamide, N,N-dimethylacetamide or N-methylpyrrolidin-2-one, an ester solvent such as ethyl acetate, acetic acid, hydrochloric acid, water, a mixed solvent thereof and the like is suitable.

Optically active amine (7a) prepared in accordance with the above-described process can be converted to optically active compound (1) in accordance with the above-described Preparation Process 2. Antipode (1) of optically active substance (1) obtained from optically active amine (7a) may also be prepared in accordance with a similar process.

Optically active compound (1) may be prepared by separating racemic compound (1) through a column composed of an optically active carrier. It is also possible to separate intermediate (2), (4), (7), (8) or (9) for preparing racemic compound (1) through a column composed of an optically active carrier to isolate optically active intermediate (2), (4), (7), (8) or (9), and then prepare optically active compound (1) in accordance with any of Preparation Processes 1 or 2. As a process for isolating optically active compound (1), optically active intermediate (2), (4), (7), (8) or (9), a process of fractionally crystallizing a salt with an optically active carboxylic acid, or a process of fractionally crystallizing a salt with an optically active base on the contrary may be used.

[Preparation Process 5]

Compound (1) in which $T^1$ is a group -CO-CO-N(R')-, in which R' has the same meaning as defined above, can be prepared in accordance with the following scheme:

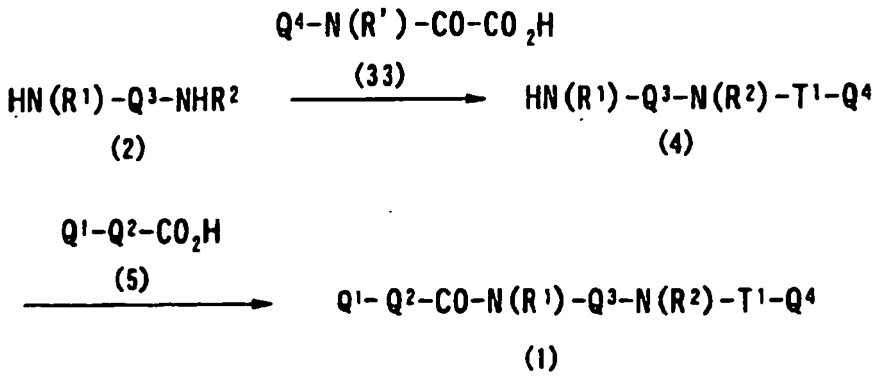

wherein $Q^1$, $Q^2$, $Q^3$, $Q^4$, $R^1$, $R^2$ and R' have the same meanings as defined above, and $T^1$ represents a group -CO-CO-N(R')-, in which R' has the same meaning as defined above.

An acid halide, activated ester or the like, which is derived from carboxylic acid (33), may react with diamine (2), giving compound (4). The resultant compound (4) may react with carboxylic acid (5) under the same conditions, giving compound (1) according to the present invention. In the above reaction steps, reagents and conditions, which are generally used in peptide synthesis, may be applied. The acid halide can be prepared by treating carboxylic acid (33) with an acid halide such as thionyl chloride or oxalyl chloride. The activated ester includes various kinds of esters. Such an ester can be prepared by, for example, reaction of a phenol such as p-nitrophenol, N-hydroxybenzotriazol, or N-hydroxysccinimide with carboxylic acid (33) using a condensing agent such as N,N'-dicyclohexylcarbodiimide or 1-ethyl-3-(3-dimethylaminopropyl)carbodiimide hydrochloride. The activated ester can also be prepared by reaction of carboxylic acid (33) with pentafluorophenyl trifluoroacetate or the like, reaction of carboxylic acid (33) with 1-benzotriazolyloxytripyrrolidinophosphonium hexafluorophosphite, reaction of carboxylic acid (33) with diethyl cyanophosphonate (Shioiri method), reaction of carboxylic acid (33) with triphenylphosphine and 2,2'-dipyridyl disulfide (Mukaiyama method) or the like. The thus-obtained mixed acid anhydride, acid halide or activated ester of carboxylic acid (33) may react with diamine (2) at -78°C to 150°C in the presence of a proper base in an inert solvent, giving compound (4). Thus-obtained compound (4) may react with a mixed acid anhydride, acid halide or activated ester of carboxylic acid (5) under the same conditions, giving compound (1) according to the present invention. The reagents and reaction conditions in the reaction of compound (4) with carboxylic acid (5) are the same as those in the reaction of diamine (2) with carboxylic acid (33). The bases and solvents used in the above respective steps may be suitably chosen from those described in Preparation Process 1.

When compound (1) in which $Q^3$ is the following group:

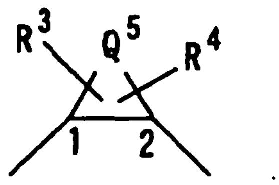

wherein $R^3$, $R^4$ and $Q^5$ have the same meanings as defined above, and numerals 1 and 2 indicate positions, and the relation between position 1 and position 2 is a trans-form or cis-form, is prepared, it is only necessary to use diamine (2a) or (2b) described in Preparation Process 3.

[Preparation Process 6]

Compound (1) in which $T^1$ is a group -CO-CO-N(R')-, in which R' has the same meaning as defined above, can also be prepared in accordance with the following scheme:

$$Q^1\text{-}Q^2\text{-}CO\text{-}N(R^1)\text{-}Q^3\text{-}HNR^2 \xrightarrow[(33)]{Q^4\text{-}N(R')\text{-}CO\text{-}CO_2H} Q^1\text{-}Q^2\text{-}CO\text{-}N(R^1)\text{-}Q^3\text{-}N(R^2)\text{-}T^1\text{-}Q^4$$

(9) (1)

wherein $Q^1$, $Q^2$, $Q^3$, $Q^9$, $R^1$, $R^2$ and R' have the same meanings as defined above, and $T^1$ represents a group -CO-CO-N(R')-, in which R' has the same meaning as defined above.

In the reaction of amine (9) with carboxylic acid (33), the same reagents and conditions as those described in Preparation Process 1 may be used.

Amine (9) used herein can also be prepared in accordance with the following scheme shown as a preparation scheme of amine (41) in addition of the scheme described in Preparation Process 2.

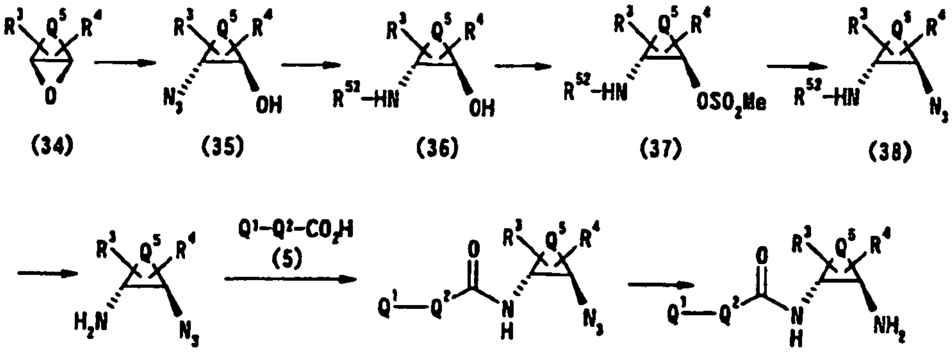


(34) (35) (36) (37) (38)

(39) (40) (41)

wherein $R^3$, $R^4$, $Q^1$, $Q^2$ and $Q^5$ have the same meanings as defined above, and $R^{52}$ represents a protecting group for amino group.

Compound (34) in the above preparation scheme can be prepared by treating a cycloalkene with perbenzoic acid or a derivative thereof in a solvent such as methylene chloride to epoxidate it. Ordinary conditions for epoxidation of an alkene may be applied to the conditions of this reaction. Compound (34) can also be prepared in accordance with the process described in J. Org. Chem., Vol. 61, pp. 8687-8691 (1996) or a process corresponding thereto.

Compound (34) may react with sodium azide in accordance with a method known *per se* in the art, giving azide (35). Azide (35) may be catalytically reduced, and the amino group of the resultant compound may be protected, giving compound (36). As examples of the protecting group for amino group in this reaction, may be mentioned those described in Preparation Process 2. Compound (36) may be converted into azide (38) in a similar manner to the process described Preparation Process 3, and the protecting group for the amino group thereof may be left, giving compound (39). Compound (39) may react with carboxylic acid (5), giving compound (40). The compound (40) may then be catalytically reduced, giving compound (41).

[Preparation Process 7]

Compound (1) in which $T^1$ is a group -CO-CO-N(R')-, in which R' has the same meaning as defined above, can also be prepared by changing the reaction of compound (9) with carboxylic acid (3) in the scheme described in Preparation Process 2 to a reaction of compound (9) with compound (33).

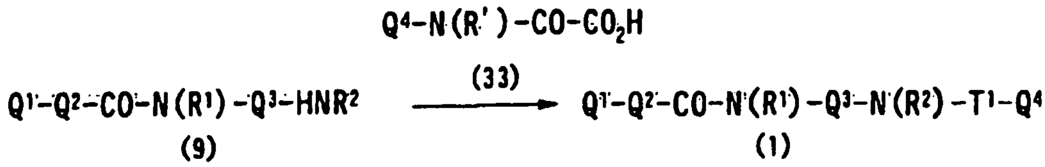

(9) (1)

wherein $Q^1$, $Q^2$, $Q^3$, $Q^4$, $R^1$, $R^2$ and R' have the same meanings as defined above, and $T^1$ represents a group -CO-CO-N(R')-, in which R' has the same meaning as defined above.

As the reaction conditions, may be applied those described in Preparation Process 2.

The important intermediates described in Preparation Process 1 to 7 of the compounds (1) will hereinafter be described.

1) The compounds described in Preparation Process 1 and 5 and represented by the following general formula (4):

$$HN(R^1)\text{-}Q^3\text{-}N(R^2)\text{-}T^1\text{-}Q^4 \qquad (4)$$

wherein $R^1$, $R^2$, $Q^3$ and $Q^4$ have the same meanings as defined above, and $T^1$ represents a group -CO-CO-N (R'), in which R' has the same meaning as defined above, are important as intermediates for preparing compounds (1).

2) The compounds described in Preparation Process 2 and 6 and represented by the following general formula (9):

$$Q^1\text{-}Q^2\text{-}C(=O)\text{-}N(R^1)\text{-}Q^3\text{-}NHR^2 \qquad (9)$$

wherein $R^1$, $R^2$, $Q^1$, $Q^2$ and $Q^3$ have the same meanings as defined above, are important as intermediates for preparing compounds (1).

5) The following optically active compounds (7a) described in Preparation Process 4 are important as intermediates for preparing compounds (1).

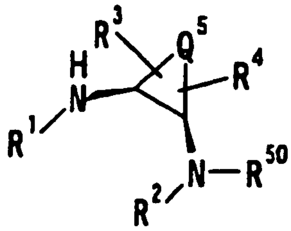

(7a)

wherein $Q^5$, $R^1$, $R^2$, $R^3$ and $R^4$ have the same meanings as defined above, and $R^{50}$ represents a protecting group for amino group.

The diamine derivatives described herein exhibit strong inhibitory effects on activated blood coagulation factor X and are thus useful for medicines for mammal including human, anticoagulants, agents for preventing and/or treating thrombosis or embolism, agents for preventing and/or treating thrombtic diseases, and agents for preventing and/or treating cerebral infarction, cerebral embolism, myocardial infarction, angina pectoris, pulmonary infarction, pulmonary embolism, Buerger's disease, deep venous thrombosis, disseminated intravascular coagulation syndrome, thrombus formation after valve or joint replacement, thrombus formation and reocclusion after angioplasty, systemic inflammatory reaction syndrome (SIRS), multiple organ disease syndrome (MODS), thrombus formation during extracorporeal circulation, or blood clotting upon blood gathering.

When a compound as described herein is used as a medicine for human body, the dose is within a range of 1 mg to 1 g, preferably 10 to 300 mg, per day for an adult. The dose for animal varies according to the object (treatment or prevention) of the administration, the kind and size of an animal to be treated, the kind of a contagium, and the condition of a disease attacked. However, it is generally within a range of 0.1 to 200 mg, preferably 0.5 to 100 mg, per

kg of weight a day. Meanwhile, the administration may be once per day, or may be divided into 2 to 4 times per day. The dose per day may exceed the above range if necessary.

Medicinal compositions comprising the compound according to the present invention can be prepared by selecting a suitable preparation form according to an administration method in accordance with a preparation method for the preparation form used. As examples of the preparation forms of the medicinal compositions comprising the compound according to the present invention as a main component, may be mentioned tablets, tablets, powder, granules, capsules, solutions, syrups, elixirs, oil or aqueous suspensions for oral preparations.

In the case of an injection, a stabilizer, a preservative and a dissolution aid may be used in a preparation. A solution which may contain these auxiliaries in some cases may also be provided as a solid form for preparing upon use by containing the solution into a container and then drying the solution by lyophilization or the like. A dose or doses of the injection may also be contained into a container.

As example of preparation forms for external application, may be mentions solutions, suspensions, emulsions, ointments, gel, creams, lotions, sprays and plasters.

A solid preparation may contain pharmaceutically acceptable additives in addition to the compound according to the present invention. For example, fillers, extenders, binders, disintegrators, dissolution accelerators, wetting agents, etc. may be suitably selected and mixed, giving a preparation.

As example of preparation forms of a liquid preparation, may be mentioned solutions, suspensions and emulsions. They may contain a suspending agent, emulsifier and/or the like in some cases.

The present invention will hereinafter be described by the following Referential Examples, Example and Test Examples. However, the present invention is not limited to the example.

[Referential Example 1]

tert-Butyl pyridin-4-ylcarbamate:

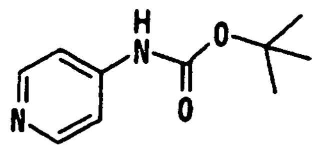

4-Aminopyridine (10 g) was dissolved in tetrahydrofuran (500 ml), di-tert-butyl dicarbonate (25.5 g) was added to the solution, and the mixture was stirred at room temperature for 10 minutes. The resultant reaction mixture was concentrated under reduced pressure, and deposited solids were washed with hexane to obtain the title compound (16.9 g).

$^1$H-NMR ($CDCl_3$) δ: 1.53(9H, s), 6.86 (1H,br.s), 7.30 (2H, dd, J=1.5, 4.9Hz) , 8.49 (2H, dd, J=1.5, 4.9Hz).

MS (FAB) m/z: 195$(M+H)^+$.

[Referential Example 2]

tert-Butyl 3-sulfanylpyridin-4-ylcarbamate:

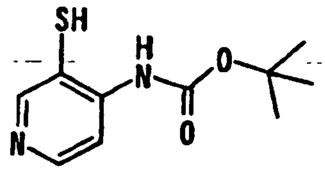

The compound (61.6 g) obtained in Referential Example 1 was dissolved in tetrahydrofuran (2,000 ml), and

the solution was stirred at -78°C for 10 minutes. A hexane solution (1.59 mol/l, 500 ml) of n-butyllithium was added dropwise to the solution, and the mixture was stirred for 10 minutes and then for 2 hours with ice cooling. After the reaction mixture was cooled to -78°C, sulfur powder (12.2 g) was added, and the resultant mixture was warmed to room temperature and stirred for 1 hour. Water (1,000 ml) was added to the reaction mixture to separate a water layer. After 3N hydrochloric acid was added to the water layer to adjust the pH of the water layer to 3 to 4, methylene chloride was added to separate an organic layer. The organic layer was dried over anhydrous sodium sulfate, and the solvent was distilled off under reduced pressure. The residue was purified by column chromatography on silica gel (methylene chloride:methanol = 50:1) to obtain the title compound (33.2 g).
$^1$H-NMR (DMSO-$d_6$) δ: 1.52 (9H, s), 7.89 (1H, d, J=6. 9Hz) , 7.99 (1H, d, J=6.4Hz), 8.20 (1H, s), 9.91 (1H, br.s).
MS (FAB) m/z: 227 $(M+H)^+$.

[Referential Example 3] Thiazolo[5,4-c]pyridine:

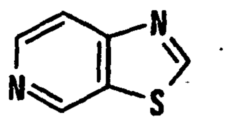

The compound (33.2 g) obtained in Referential Example 2 was dissolved in formic acid (250 ml), and the solution was heated under reflux for 3 days. The reaction mixture was concentrated under reduced pressure, and a 5N aqueous solution (100 ml) of potassium hydroxide and diethyl ether were added to the residue to separate an organic layer. The organic layer was dried over anhydrous sodium sulfate, and the solvent was then distilled off under reduced pressure. The residue was purified by column chromatography on silica gel (methylene chloride:methanol = 25:1) to obtain the title compound (9.03 g).
$^1$H-NMR ($CDCl_3$) δ: 8.05 (1H, d, J=5.9Hz) , 8.70 (1H, d, J=5.9Hz) , 9.23 (1H, s), 9.34 (1H, s).
MS (FAB) m/z: 137 $(M+H)^+$.

[Referential Example 4]

5-Methyl-4,5,6,7-tetrahydrothiazolo[5,4-c]pyridine:

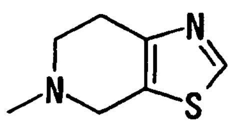

The compound (1.61 g) obtained in Referential Example 3 was dissolved in N,N-dimethylformamide (50 ml), and to the solution methyl iodide (1.50 ml) was added, the resultant mixture was stirred at 80°C for 4 hours. The reaction mixture was concentrated under reduced pressure, and the residue was dissolved in methanol (100 ml), sodium borohydride (1.53 g) was added, and the resultant mixture was stirred at room temperature for 1 hour. The reaction mixture was concentrated under reduced pressure, and a saturated aqueous solution of potassium carbonate and diethyl ether were added to the residue to separate an organic layer. The organic layer was dried over anhydrous sodium sulfate, and the solvent was distilled off under reduced pressure. The residue was purified by column chromatography on silica gel (methylene chloride:methanol = 25:1) to obtain the title compound (1.28 g).
$^1$H-NMR ($CDCl_3$) δ: 2.52(3H,s), 2.83 (2H, t, J=5.9Hz), 2.98 (2H, t, J=5.9Hz) , 3.70 (2H, s) , 8.63 (1H, s).
MS (FAB) m/z: 155 $(M+H)^+$.

[Referential Example 5]

Lithium 5-methyl-4,5,6,7-tetrahydrothiazolo[5,4-c]-pyridine-2-carboxylate:

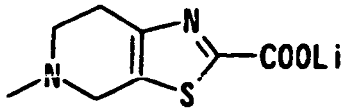

The compound (6.43 g) obtained in Referential Example 4 was dissolved in absolute tetrahydrofuran (200 ml), to the soltion n-butyllithium (1.47N hexane solution, 34.0 ml) was added dropwise at -78°C, and the resultant mixture was stirred for 40 minutes. After carbon dioxide gas was blown into the reaction mixture at -78°C for 1 hour, the reaction mixture was warmed to room temperature and then concentrated under reduced pressure to obtain the title compound (9.42 g).
$^1$H-NMR (DMSO-$d_6$) δ: 2.37 (3H, s), 2.64-2.77 (4H,m) , 3.54(2H,s).
MS (FAB) m/z: 199(M+H)$^+$.

[Referential Example 6]

tert-Butyl 2-amino-6,7-dihydrothiazolo[5,4-c]pyridine-5[4H]-carboxylate:

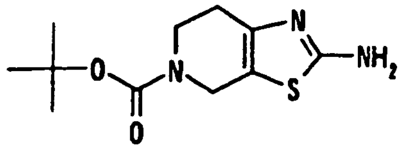

1-tert-Butoxycarbonyl-4-piperidone (40.0 g) was dissolved in cyclohexane (80 ml), and to the solution p-toluenesulfonic acid monohydrate (191 mg) and pyrrolidine (17.6 ml) were added. The mixture was heated under reflux for 2 hours while removing water using a Dean-Stark trap. After the reaction mixture was concentrated under reduced pressure, the residue was dissolved in methanol (60 ml), and sulfur powder (6.42 g) was added. A methanol solution (10 ml) of cyanamide (8.44 g) was slowly added dropwise to the solution with ice cooling, and the mixture was stirred at room temperature for 5 hours. Precipitated solid materials were collected by filtration to obtain the title compound (31.0 g).
$^1$H-NMR (DMSO-$d_6$) δ: 1.41 (9H, s), 2.44 (2H, t, J=5.6Hz), 3.57 (2H, t, J=5.6Hz), 4.29 (2H, s), 6.79(2H,s).
MS (EI) m/z: 255(M$^+$).

[Referential Example 7]

tert-Butyl 2-bromo-6,7-dihydrothiazolo[5,4-c]pyridine-5[4H]-carboxylate:

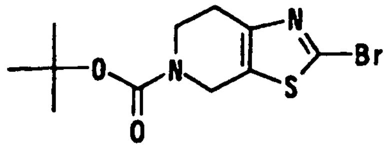

Copper(II) bromide (1.05 g) was suspended in N,N-dimethylformamide(20 ml), and tert-butyl nitrite (0.696 ml) and the compound (1.00 g) obtained in Referential Example 6 were added with ice cooling, the reaction mixture was heated and stirred at 40°C for 30 minutes. The reaction mixture was concentrated under reduced pressure, and the residue was purified by column chromatography on silica gel (ethyl acetate:hexane = 1:5) to obtain the title compound (568 mg).
$^1$H-NMR ($CDCl_3$) δ: 1.48 (9H, s), 2.85(2H,br.s), 3.72(2H,br.s), 4.56(2H,br.s).
MS (FAB) m/z: 319(M+H)$^+$.

[Referential Example 8]

2-Bromo-4,5,6,7-tetrahydrothiazolo[5,4-c]pyridine trifluoroacetate:

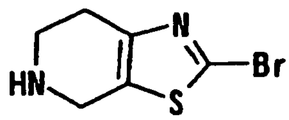

The compound (890 mg) obtained in Referential Example 7 was dissolved in methylene chloride (2 ml), and to the solution trifluoroacetic acid (15 ml) was added, and the mixture was stirred at room temperature for 30 seconds. The reaction mixture was concentrated under reduced pressure, and diethyl ether was added to the residue. Precipitated solid materials were collected by filtration to obtain the title compound (867 mg).
$^1$H-NMR (DMSO-$d_6$) δ: 2.98(2H,t,J=6.1Hz), 3.45 (2H, t, J=6.1Hz), 4.35 (2H, s) , 9.53 (2H, br.s).
MS (FAB) m/z: 219 $(M+H)^+$.

[Referential Example 9]

2-Bromo-5-methyl-4,5,6,7-tetrahydrothiazolo[5,4-c]-pyridine:

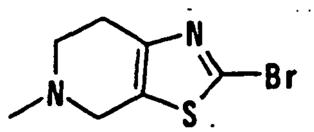

The compound (422 mg) obtained in Referential Example 8 was suspended in methylene chloride (10 ml), and triethylamine (0.356 ml) was added to make a solution. Acetic acid (0.216 ml), an aqueous solution (35% solution, 0.202 ml) of formaldehyde and sodium triacetoxyborohydride (428 mg) were successively added to the solution, and the resultant mixture was stirred at room temperature for 1 hour. A saturated aqueous solution (100 ml) of sodium hydrogencarbonate, methylene chloride (100 ml) and a 3N aqueous solution (3 ml) of sodium hydroxide were added to the reaction mixture to conduct liquid separation. After an organic layer was dried over anhydrous sodium sulfate, the solvent was distilled off under reduced pressure. The residue was purified by column chromatography on silica gel (methylene chloride:methanol = 100:3) to obtain the title compound (286 mg).
$^1$H-NMR ($CDCl_3$) δ: 2.49 (3H, s), 2.79 (2H, t, J=5.7Hz), 2.85-2.93(2H,m), 3.58 (2H, t, J=1.8Hz).
MS (FAB) m/z: 233 $(M+H)^+$.

[Referential Example 10]

Lithium 5-methyl-4,5,6,7-tetrahydrothiazolo[5,4-c]-pyridine-2-carboxylate:

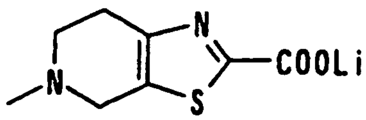

The compound (531 mg) obtained in Referential Example 9 was dissolved in absolute diethyl ether (20 ml), n-butyllithium (1.54N hexane solution, 1.63 ml) was added dropwise at -78°C, and the mixture was stirred for 30 minutes

with ice cooling. After passing carbon dioxide into the reaction mixture at -78°C for 10 minutes, the mixture was warmed to room temperature. The reaction mixture was concentrated under reduced pressure to obtain the title compound (523 mg).
$^1$H-NMR (DMSO-$d_6$) δ: 2.37 (3H, s), 2.64-2. 85 (4H, m), 3.54 (2H, s).

[Referential Example 11]

Ethyl (1R*,3R*,6S*)-7-oxabicyclo[4.1.0]heptane-3-carbooxylate:

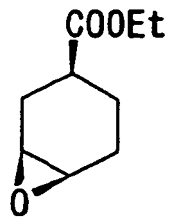

(1R*,4R*,5R*)-4-Iodo-6-oxabicyclo[3.2.1]octan-7-one (J. Org. Chem., 1996, Vol. 61, p. 8687) (14.3 g) was dissolved in ethanol (130 ml), a 2N aqueous solution (34.5 ml) of sodium hydroxide was added under ice cooling, and the mixture was then stirred at room temperature for 7 hours. After the solvent was distilled off under reduced pressure, and water was added to the residue to conduct extraction with methylene chloride, the extract was dried over anhydrous sodium sulfate. The solvent was distilled off under reduced pressure, and the residue was purified by column chromatography on silica gel (hexane:ethyl acetate = 83:17) to obtain the title compound (6.54 g).
$^1$H-NMR ($CDCl_3$) δ: 1.25 (3H, t, J=7.1Hz), 1.50-1.70 (2H, m), 1.71-1.82 (1H, m), 2.08-2.28(4H,m), 3.16(2H,s), 4.12 (2H, q, J=7.1Hz).

[Referential Example 12]

Ethyl (1R*,3S*,4S*)-3-azido-4-hydroxycyclohexane-carboxylate:

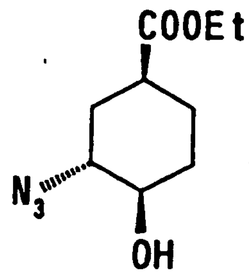

The compound (13.6 g) obtained in Referential Example 11 was dissolved in N,N-dimethylformamide (100 ml), ammonium chloride (6.45 g) and sodium azide (7.8 g) were successively added at room temperature, and the mixture was then stirred at 75°C for 12 hours. The solvent was concentrated to about 1/3, and the residue was diluted with water and ethyl acetate to conduct stirring for 3 minutes. The resultant organic layer was washed with water and saturated aqueous solution of sodium chloride and dried over anhydrous magnesium sulfate. The solvent was distilled off under reduced pressure, and the residue was purified by column chromatography on silica gel (ethyl acetate:hexane = 1:4) to obtain the title compound (15.8 g).
$^1$H-NMR ($CDCl_3$) δ: 1.28 (3H, t, J=7.1Hz), 1.37-1.67 (2H, m), 1.86-1.95 (1H, m), 2.04-2.18(2H,m), 2.32-2.43 (1H, m), 2.68-2.78 (1H, m), 3.40-3.60 (2H, m), 4.17 (2H, q, J=7.1Hz).

[Referential Example 13]

Ethyl (1R*,3S*,4S*)-3-[(tert-butoxycarbonyl)amino]]-4-hydroxycyclohexanecarboxylate:

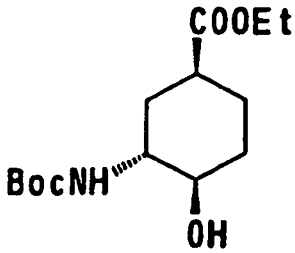

The compound (100 mg) obtained in Referential Example 12 and di-tert-butyl dicarbonate (133 mg) were dissolved in ethyl acetate (12 ml) and a catalytic amount of 10% palladium on carbon was added to stir the mixture at room temperature for 12 hours in a hydrogen atmosphere. After insoluble matter was removed by filtration, the solvent was distilled off under reduced pressure, and the residue was purified by column chromatography on silica gel (hexane: ethyl acetate = 3:1) to obtain the title compound (145 mg).
$^1$H-NMR ($CDCl_3$) δ: 1.28(3H,t,J=7.1Hz), 1.45 (9H,s), 1.38-1.57(2H,m), 1.86-1.95(1H,m), 2.05-2.17(1H,m), 2.29-2.39 (2H,m), 2.61-2.68(1H,m), 3.25-3.66(3H,m), 9.17(2H,q,J=7.1Hz), 4.53(1H,br.s).

[Referential Example 14]

Ethyl (1R*,3S*,4R*)-4-azido-3-[(tert-butoxycarbonyl)amino]-cyclohexanecarboxylate and ethyl (1R*,3S*,4S*)-4-azido-3-[(tert-butoxycarbonyl)amino]cyclohexanecarboxylate:

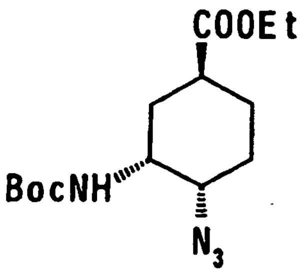 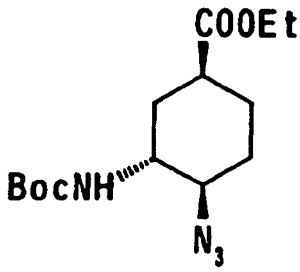

After the compound (16 g) obtained in Referential Example 13 and triethylamine (38 ml) were dissolved in methylene chloride (150 ml), and the solution was cooled to -78°C, methanesulfonyl chloride (13 ml) was added dropwise at the same temperature. After stirring for 15 minutes at the same temperature, the mixture was heated to 0°C and stirred for 30 minutes and then 2 hours at room temperature. After 0.1N hydrochloric acid was added, and the mixture was diluted with methylene chloride, the resultant organic layer was separated, washed with a saturated aqueous solution of sodium hydrogencarbonate and saturated aqueous solution of sodium chloride and dried over anhydrous magnesium sulfate. The solvent was distilled off under reduced pressure to obtain crude ethyl (1R*,3S*,4S*)-3-[(tert-butoxycarbonyl) amino]-4-[(methanesulfonyl)oxy]cyclohexane-carboxylate.
The product obtained above was dissolved in N,N-dimethylformamide (100 ml), and sodium azide (18 g) was added at room temperature. The mixture was heated to 75°C and stirred for 12 hours. The solvent was concentrated to about 1/3, and the residue was diluted with water and ethyl acetate to conduct stirring for 3 minutes. The resultant organic layer was separated, washed with saturated aqueous solution of sodium chloride and dried over anhydrous magnesium sulfate. The solvent was distilled off under reduced pressure, and the residue was purified by column chromatography on silica gel (ethyl acetate:hexane = 1:4) to obtain the title compounds [(1R*,35*,4R*)-form (6.74 g) and (1R*,3S*,4S*)-form (1.32 g)].
(1R*,3S*,4R*)-form:

$^1$H-NMR ($CDCl_3$) δ: 1.26(3H,t,J=7.1Hz), 1.95(9H,s), 1.38-2.33(6H,m), 2.57-2.68(1H,m), 3.77-4.20(4H,m), 4.63(1H, br.s).

(1R*,3S*,4S*)-form:

$^1$H-NMR ($CDCl_3$) δ: 1.27(3H,t,J=7.1Hz), 1.46(9H,s), 1.53-2.30(6H,m), 2.50-2.65(1H,m), 3.42-3.72(2H,m), 4.15(2H, q.J=7.1Hz), 4.67(1H,br.s).

[Referential Example 15]

Ethyl (1R*,3S*,4R*)-4-amino-3-[(tert-butoxycarbonyl)-amino]cyclohexanecarboxylate:

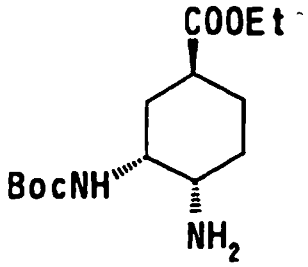

Ethyl (1R*,3S*,4R*)-4-azido-3-[(tert-butoxycarbonyl)amino]cyclohexanecarboxylate (5.4 g) obtained in Referential Example 14 was dissolved in a mixed solvent of ethanol (10 ml) and ethyl acetate (10 ml), and a catalytic amount of 10% palladium on carbon was added to stir the mixture at room temperature for 20 hours in a hydrogen atmosphere. After insoluble matter was removed by filtration, the solvent was distilled off under reduced pressure to obtain the title compound (4.7 g).

[Referential Example 16]

Ethyl (1R*,3S*,4R*)-3-[(tert-butoxycarbonyl)amino]-4-{[(5-chloroindol-2-yl)carbonyl]amino}cyclohexanecarboxylate:

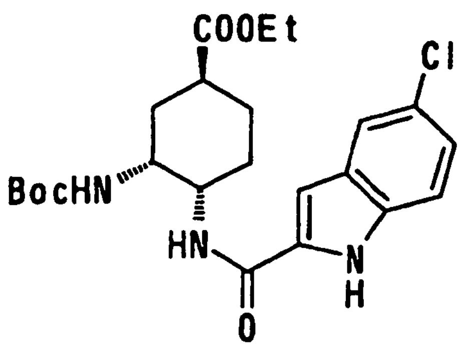

The compound (4.62 g) obtained in Referential Example 15] was dissolved in methylene chloride (50 ml), 5-chloroindole-2-carboxylic acid (3.63 g), 1-hydroxybenzotriazole monohydrate (2.43 g) and 1-(3-dimethylaminopropyl)-3-ethylcarbodiimide hydrochloride (3.45 g) were added at room temperature, and the mixture was stirred for 12 hours. After 0.1N hydrochloric acid was added, and the mixture was extracted with methylene chloride, the resultant organic layer was washed with a saturated aqueous solution of sodium hydrogencarbonate and saturated aqueous solution of

sodium chloride and dried over anhydrous magnesium sulfate. The solvent was distilled off under reduced pressure, and the residue was purified by column chromatography on silica gel (ethyl acetate:hexane = 2:3) to obtain the title compound (5.3 g). $^1$H-NMR ($CDCl_3$) δ: 1.26(3H,t,J=7.1Hz), 1.43(9H,s), 1.35-2.46(7H,m), 3.91-4.02(1H,m), 4.10-4.22 (2H,m), 4.79(1H,br.s), 6.79(1H,s), 7.18-7.40(2H,m), 7.59(1H,s), 8.00(1H,br.s), 9.13(1H,br.s).

[Referential Example 17]

Ethyl (1S,3S,6R)-7-oxabicyclo[4.1.0]heptane-3-carboxylate:

(1S,4S,5S)-4-Iodo-6-oxabicyclo[3.2.1]octan-7-one (J. Org. Chem., 1996, Vol. 61, p. 8687) (89.3 g) was suspended in ethanol (810 ml), a 2N aqueous solution (213 ml) of sodium hydroxide was added, and the mixture was then stirred at room temperature for 3 hours. After the solvent was distilled off under reduced pressure, and water was added to the residue to conduct extraction with methylene chloride, the extract was dried over anhydrous magnesium sulfate. The solvent was distilled off under reduced pressure, and the residue was purified by column chromatography on silica gel (hexane:ethyl acetate = 17:3) to obtain the title compound (41.3 g).
$[\alpha]_D^{25}$ = 58° (C=1.0, hloroform).

[Referential Example 18]

Ethyl (1S,3R,4R)-3-azido-4-hydroxycyclohexanecarboxylate:

The compound (41 g) obtained in Referential Example 17 was dissolved in N,N-dimethylformamide (300 ml), ammonium chloride (19.3 g) and sodium azide (23.5 g) were successively added at room temperature, and the mixture was then stirred at 76°C for 13 hours. The reaction mixture was filtered, the filtrate was concentrated, the product previously captured by the filter was put in the residue, and water was added to dissolve the collected product. The solution was extracted with ethyl acetate. The resultant organic layer was washed with water and saturated aqueous-solution of sodium chloride and then dried over anhydrous magnesium sulfate. The solvent was distilled off under reduced pressure to obtain the title compound (51.5 g).
$[\alpha]_D^{25}$ = +8° (C=1.0, chloroform).

[Referential Example 19]

Ethyl (1S,3R,4R)-3-[(tert-butoxycarbonyl)amino]-4-hydroxycyclohexanecarboxylate:

The 10 compound (51.2 g) obtained in Referential Example 18 and di-tert-butyl dicarbonate (68.1 g) were dissolved in ethyl acetate (1000 ml), 5% palladium on carbon (5.0 g) was added, and the mixture was stirred overnight at room temperature under a hydrogen pressure of 7 kg/cm$^2$. After insoluble matter was removed by filtration, the solvent was distilled off under reduced pressure, the residue was purified by column chromatography on silica gel (hexane:ethyl acetate = 4:1 → 3:1), and hexane was added to solidify it to obtain the title compound (46.9 g). $[\alpha]_D^{25}$ = +25° (C=1.0, chloroform).

[Referential Example 20]

Ethyl (1S,3R,4S)-4-azido-3-[(tert-butoxycarbonyl)amino]-cyclohexanecarboxylate and ethyl (1S,3R,4R)-4-azido-3-[(tert-butoxycarbonyl)amino]cyclohexanecarboxylate:

The compound (53.5 g) obtained in Referential Example 19 and triethylamine (130 ml) were dissolved in methylene chloride (500 ml), and methanesulfonyl chloride (42 ml) was added dropwise over 20 minutes under cooling at -10°C to -15°C. After stirring for 20 minutes at the same temperature, the mixture was heated to room temperature over 2 hours. The reaction mixture was cooled to 0°C, 0.5N hydrochloric acid (800 ml) was added dropwise, and the mixture was extracted with methylene chloride. The resultant organic layer was washed with a saturated aqueous solution of sodium hydrogencarbonate and saturated aqueous solution of sodium chloride and dried over anhydrous magnesium sulfate. The solvent was distilled off under reduced pressure to obtain crude ethyl (1S,3R,4R)-3-[(tert-butoxycarbonyl) amino]-4-[(methylsulfonyl)oxy]cyclohexanecarboxylate.

The crude product obtained above was dissolved in N,N-dimethylformamide (335 ml), and sodium azide (60.5 g) was added to stir the mixture at 67°C to 75°C for 16 hours. The reaction mixture was filtered, the filtrate was concentrated to distill off 250 ml of the solvent, the product captured by the filter was put in the residue, and the collected product was dissolved in water and extracted with ethyl acetate. The resultant organic layer was washed with saturated aqueous

solution of sodium chloride and dried over anhydrous magnesium sulfate. The solvent was distilled off under reduced pressure, and the residue was purified by column chromatography on silica gel (ethyl acetate:hexane = 1:4) to obtain the title compounds [(1S,3R,4S)-form (18.4 g) and (1S,3R,4R)-form (3.3 g)]. (1S,3R,4S)-form: $[\alpha]_D^{25}$ = +62° (C=1.0, chloroform). (1S,3R,4R)-form: $[\alpha]_D^{25}$ = -19° (C=1.0, chloroform).

[Referential Example 21]

Ethyl (1S,3R,4S)-9-Amino-3-[(tert-butoxycarbonyl)amino] cyclohexanecarboxylate:

The compound (4.0 g) obtained in Referential Example 20 was dissolved in a mixed solvent of ethanol (150 ml) and ethyl acetate (150 ml), and 5% palladium on carbon (0.5 g) was added to stir the mixture at room temperature for 17 hours in a hydrogen atmosphere (5 kg/cm$^2$). After insoluble matter was removed by filtration, the solvent was distilled off under reduced pressure to obtain the title compound (4.2 g).

[Referential Example 22]

Ethyl (1S,3R,4S)-3-[(tert-butoxycarbonyl)amino]-4-{[(5-chloroindol-2-yl)carbonyl]amino}cyclohexanecarboxylate:

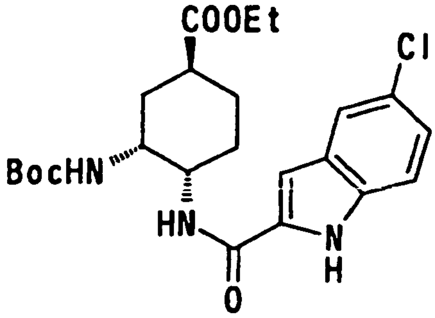

The compound (4.2 g) obtained in Referential Example 21 was dissolved in methylene chloride (50 ml), 5-chloroindole-2-carboxylic acid (3.33 g), 1-hydroxybenzotriazole monohydrate (2.52 g) and 1-(3-dimethylaminopropyl)-3-ethylcarbodiimide hydrochloride (3.15 g) were added at room temperature, and the mixture was stirred for 12 hours. After 0.1N hydrochloric acid was added to the reaction mixture, and the mixture was extracted with methylene chloride, the resultant organic layer was washed with a saturated aqueous solution of sodium hydrogencarbonate and saturated aqueous solution of sodium chloride and dried over anhydrous magnesium sulfate. The solvent was distilled off under reduced pressure, and the residue was purified by column chromatography on silica gel (ethyl acetate:hexane = 1:1) to obtain the title compound (4.36 g).
$[\alpha]_D^{25}$ = -27° (C=1.0, chloroform).

[Referential Example 23]

Ethyl (1S,3R,4S)-4-{[benzyloxy)carbonyl]amino}-3-[(tert-butoxycarbonyl)amino]cyclohexanecarboxylate:

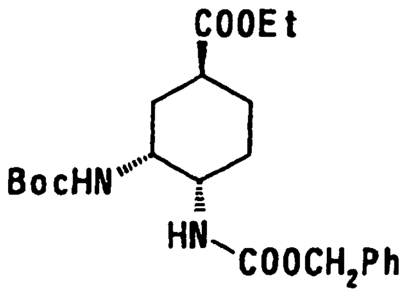

The compound (3.10 g) obtained in Referential Example 21 was dissolved in tetrahydrofuran (50 ml), and a saturated aqueous solution (50 ml) of sodium hydrogencarbonate was added. After benzyloxycarbonyl chloride (1.71 ml) was added dropwise to the reaction mixture under ice cooling, the mixture was stirred at room temperature for 4 days. Ethyl acetate (200 ml) and water (200 ml) were added to the reaction mixture to conduct liquid separation. After the resultant organic layer was dried over anhydrous sodium sulfate, the solvent was distilled off under reduced pressure. Solids deposited were collected by filtration to obtain the title compound (3.24 g).
$^1$H-NMR ($CDCl_3$ δ: 1.24(3H,t,J=7.1Hz), 1.29-1.44(1H,m), 1.44(9H,s), 1.51-1.64(1H,m), 1.72-2.10(4H,m), 2.27-2.43(1H, m), 3.60-3.73(1H, m), 4.00-4.18(3H, m), 4.62(1H,br.s), 5.01-5.13(2H,m), 5.26(1H, br.s), 7.27-7.38(5H, m).

[Referential Example 24]

(1S,3R,4S)-4-{[(Benzyloxy)carbonyl]3mino}-3-[(tert-butoxycarbonyl)amino] cyclohexanecarboxylic acid:

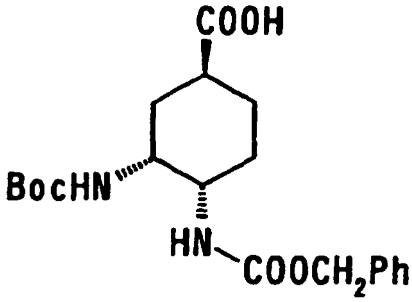

The compound (620 mg) obtained in Referential Example 23 was dissolved in tetrahydrofuran (20 ml), and an aqueous solution (10 ml) of lithium hydroxide monohydrate (93 mg) was added to stir the mixture at room temperature for 16 hours. After lithium hydroxide monohydrate (217 mg) was additionally added to the reaction mixture, and the mixture was stirred at room temperature for 2 hours, the reaction mixture was neutralized with 1N hydrochloric acid and extracted with methylene chloride. An organic layer was washed with saturated aqueous solution of sodium chloride and then dried over anhydrous sodium sulfate. The solvent was distilled off under reduced pressure to obtain the title compound (600 mg).
$^1$H-NMR ($CDCl_3$) δ: 1.22-2.20(6H, m), 1.44(9H,s), 2.45(1H,br.s), 3.60-3.80(1H,br), 4.09(1H,br.s), 4.66 (1H,br.s), 5.00-5.20(2H,m), 5.26(1H,br.s), 7.20-7.40(5H,m).
MS (ESI) m/z: 393$(M+H)^+$.

[Referential Example 25]

Benzyl (1S,2R,4S)-2-[(tert-butoxycarbonyl)amino]-4-[(dimethylamino)carbonyl]cyclohexylcarbamate:

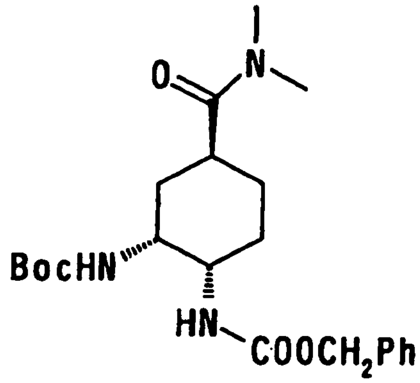

After the compound (600 mg) obtained in Referential Example 24 and dimethylamine hydrochloride (240 mg) were suspended in methylene chloride (50 ml), a proper amount of tetrahydrofuran was added to the suspension to prepare a solution. To this solution were added triethylamine (0.41 ml), 1-(3-dimethylaminopropyl)-3-ethylcarbodiimide hydrochloride (422 mg) and 1-hydroxybenzotriazole monohydrate (338 mg), and the mixture was stirred at room temperature for 1 hour. Dimethylamine hydrochloride (480 mg) and triethylamine (0.82 ml) were additionally added to the reaction mixture to stir the mixture at room temperature for additional 18 hours. The reaction mixture was poured into water to separate an organic layer. After the organic layer was washed with 1N hydrochloric acid and saturated aqueous solution of sodium chloride and dried over anhydrous sodium sulfate. The solvent was distilled off under reduced pressure, and the residue was purified by column chromatography on silica gel (methanol:methylene chloride = 3:47 → 2:23) to obtain the title compound (620 mg).
$^1$H-NMR ($CDCl_3$) δ: 1.20-1.50(2H,m), 1.44(9H,s), 1.50-2.10(4H,m), 2.60(1H,br.t,J=11.6Hz), 2.93(3H,s), 3.02(3H,s), 3.70 (1H,br.s), 4.14(1H,br.s), 4.65(1H,br.s), 5.00-5.30(3H,m), 7.26-7.40(5H,m).
MS (ESI) m/z = 420(M+H)$^+$.

[Referential Example 26]

tert-Butyl (1R,2S,5S)-2-amino-5-[(dimethylamino)-carbonyl]cyclohexylcarbamate:

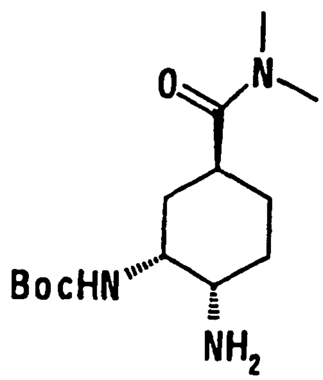

10% Palladium on carbon (57 g) was added to a solution of the compound (190 g) obtained in Referential Example 143 in methanol (8000 ml), and the mixture was stirred for 3 hours under a hydrogen pressure (7 atm). After the catalyst was removed by filtration, the filtrate was concentrated under reduced pressure. After toluene was added to the residue, and the mixture was concentrated under reduced pressure, hexane (2500 ml) was added to solidify a product. The product was collected by filtration and dried to obtain the title compound (121 g).
$^1$H-NMR ($CDCl_3$) δ: 1.20-1.77(6H,m), 1.45(9H,s), 2.20-2.35(1H,br), 2.63-2.74(1H,m), 2.92(3H,s), 3.02(3H,s), 3.02-3.11 (2H,m), 3.74-3.82(1H,m), 4.88-5.00(1H,br).
MS (ESI) m/z: 286(M+H)$^+$.

[Referential Example 27]

tert-Butyl (1R,2S,5S)-2-[(6-chloro-2-naphthoyl)amino]-5-[(dimethylamino)carbonyl]cyclohexylcarbamate:

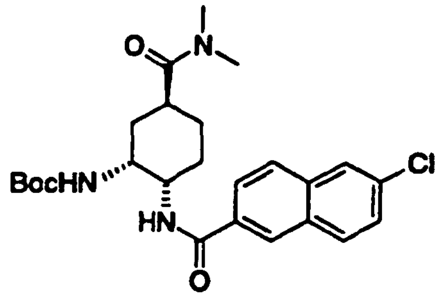

The title compound was obtained from the compound obtained in Referential Example 26 and 6-chloronaphthalene-2-carboxylic acid (Eur. J. Chem-Chim. Ther., 1984, Vol. 19, pp. 205-214) in a similar manner to Referential Example 22.
$^1$H-NMR ($CDCl_3$) δ: 1.30-2.00(15H,m), 2.60-2.80(1H,m), 2.96(3H,s), 3.09(3H,s), 4.00-4.20(1H,m), 4.20-4.30(1H,m), 4.75-4.95(1H,m), 7.44(1H,d,J=9.0Hz), 7.70-7.95(5H,m), 8.31(1H,s).
MS (FAB) m/z: $474(M+H)^+$.

[Referential Example 28]

Lithium 2-[(5-chloropyridin-2-yl)amino]-2-oxoacetate:

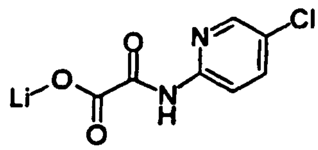

Methyl chlorooxoacetate (78.7 ml) was added dropwise to a suspension of 2-amino-5-chloropyridine (100 g) and sodium hydrogencarbonate (78.4 g) in tetrahydrofuran (2000 ml) at 0°C, and the mixture was stirred at room temperature for 2 hours. After the reaction mixture was added to a mixture of diethyl ether (2000 ml), ammonium chloride (62.4 g) and water (1000 ml), liquid separation was performed. The resultant water layer was extracted with methylene chloride. Organic layers were combined and dried over anhydrous sodium sulfate, and the solvent was distilled off under reduced pressure to obtain methyl 2-[(5-chloropyridin-2-yl)amino]-2-oxoacetate (162 g). Water (450 ml) and lithium hydroxide (18.2 g) were added to a solution of this ester (160 g) in tetrahydrofuran (1800 ml). After the mixture was stirred at room temperature for 2 hours, the solvent was distilled off under reduced pressure, and hexane (3000 ml) was added to the resultant residue to stir the mixture for 3 hours. Solids were collected by filtration and dried. Acetonitrile (1000 ml) was added to the solids (190 g), and the mixture was stirred for 1 hour. Solids formed were collected by filtration, washed with diethyl ether (500 ml) and then dried to obtain the title compound (158 g).
$^1$H-NMR (DMSO-$d_6$) δ: 7.92(1H,dd,J=9.1,2.7Hz), 8.13(1H,dd,J=9.1,0.5Hz), 8.36(1H,dd,J=2.7,0.5Hz), 10.19(1H,s).

[Referential Example 29]

Ethyl (1S,3R,4S)-4-{[(allyloxy)carbonyl]amino}-3-[(tert-butoxycarbonyl)amino]cyclohexanecarboxylate:

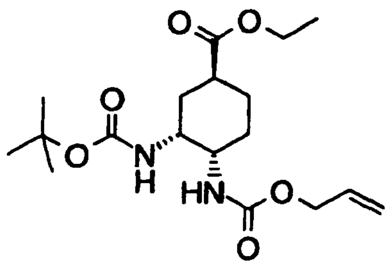

A 10% palladium on carbon catalyst (10.2 g) was added to a solution of the compound (10.0 g) obtained in Referential Example 23 in a mixed solvent of tetrahydrofuran (40 ml) and ethanol (40 ml), and the mixture was stirred at room temperature for 63 hours under a hydrogen atmosphere. After the catalyst was removed by filtration, the resultant filtrate was concentrated under reduced pressure. After the resultant colorless oil was dissolved in tetrahydrofuran (25 ml), and pyridine (2.3 ml) was added to the solution at room temperature, allylchloroformate (2.70 ml) was added dropwise at 0°C, and the mixture was stirred for 20 minutes. After ice and ethyl acetate were added to the reaction mixture, and the resultant mixture was stirred for 5 minutes, A 10% aqueous solution of citric acid was added to acidify the mixture. The resultant organic layer was washed with a saturated aqueous solution of sodium hydrogencarbonate and a saturated aqueous solution of sodium chloride, dried over anhydrous sodium sulfate and then concentrated under reduced pressure. The resultant residue was purified by column chromatagraphy on silica gel (methylene chloride:methanol = 40:1) to obtain the title compound (6.03 g).
$^1$H-NMR ($CDCl_3$) δ: 1.25(3H,t,J=7.1Hz), 1.31-1.40(1H,m), 1.45(9H,s), 1.51-1.65(1H,m), 1.72-1.86(1H,m), 1.89-2.10(3H, m), 2.25-2.50(1H,br), 3.63-3.72(1H,m), 4.03-4.15(1H,br), 4.13(2H,q,J=7.1Hz), 4.49-4.59(2H,m), 4.60-4.75(1H,m), 5.20 (1H,d,J=10.5Hz), 5.22-5.32(1H,br), 5.29(1H,dd,J=17.1,1.7Hz), 5.85-5.97(1H,m).
-MS (ESI) m/z: 371(M+H)$^+$.

[Referential Example 30]

(1S,3R,4S)-4-{[(Allyloxy)carbonyl]amino}-3-[(tert-butoxycarbonyl)amino]cyclohexanecarboxylic acid:

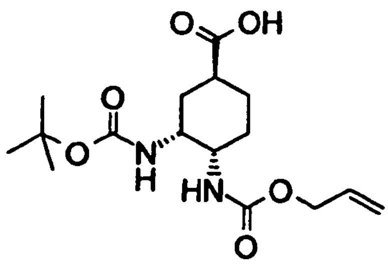

The title compound was obtained from the compound obtained in Referential Example 29 in a similar manner to the process described in Referential Example 24.
$^1$H-NMR ($CDCl_3$ δ: 1.35-2.15(6H,br), 1.45(9H,s), 2.35-2.65(1H,br), 3.65-3.75(1H,m), 4.00-4.15(1H,br), 4.48-4.63(2H, m), 4.63-4.80(1H,br), 5.03-5.33(1H,br), 5.21(1H,d,J=10.3Hz), 5.29(1H,dd,J=17.1,1.5Hz), 5.86-5.97(1H,m).
MS (ESI) m/z: 343(M+H)$^+$.

[Referential Example 31]

2,2,2-Trichloroethyl (1S,3R,4S)-4-{[(allyloxy)carbonyl]-amino}-3-[(tert-butoxycarbonyl)amino]cyclohexanecarboxylate:

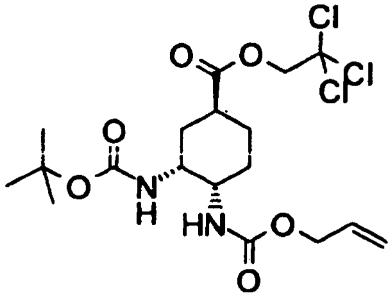

1-(3-Dimethylaminopropyl) -3-ethylcarbodiimide hydrochloride (4.99 g), 1-hydroxybenzotriazole (2.81 g), 2,2,2-trichloroethanol (4.15 ml) and 4-dimethylaminopyridine (4.15 g) were added to a solution of the compound (5.93 g) obtained in Referential Example 30 in N,N-dimethylformamide (40 ml), and the mixture was stirred at room temperature for 1.5 hours. After the reaction mixture was concentrated under reduced pressure, ethyl acetate and water were added to the residue. The resultant water layer was extracted with ethyl acetate, and organic layers were combined, washed with a 10% aqueous solution of citric acid, a saturated aqueous solution of sodium hydrogencarbonate and a saturated aqueous solution of sodium chloride, dried over anhydrous sodium sulfate and then concentrated under reduced pressure. The residue was purified by column chromatography on silica gel (methylene chloride:methanol = 40:1) to obtain the title compound (8.88 g).
$^1$H-NMR ($CDCl_3$) δ: 1.35-1.50(1H,m), 1.46(9H,s), 1.55-1.73(1H,m), 1.77-2.22(4H,m), 2.50-2.65(1H,br), 3.66-3.75(1H,m), 4.05-4.20(1H,m), 4.50-4.60(2H,m), 4.60-4.80(1H,br), 4.71(1H,d,J=11.8Hz), 4.77(1H,d,J=11.8Hz), 5.18-5.34(1H,br), 5.20(1H,d,J=10.5Hz),
5.30(1H,dd,J=17.4,1.0Hz), 5.86-5.97(1H,m).
MS (ESI) m/z: 473[(M+H)$^+$,3xCl$^{35}$), 475[(M+H)$^+$,2xCl$^{35}$,Cl$^{37}$], 477[(M+H)$^+$,Cl$^{35}$,2xCl$^{37}$].

[Referential Example 32]

2-,2,2-Trichloroethyl (1S,3R,4S)-4-amino-3-[(tert-butoxycarbonyl)amino]cyclohexanecarboxylate:

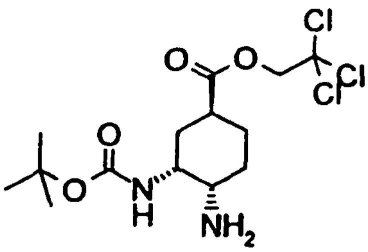

Diethylamine (20 ml) and tetrakis(triphenylphosphine)palladium (719 mg) were added to a solution of the compound (8.83 g) obtained in Referential Example 31 in tetrahydrofuran (35 ml), and the mixture was stirred at room temperature for 2.5 hours under argon. A 10% aqueous solution (250 ml) of citric acid was added to the reaction mixture to acidify it, and diethyl ether was added thereto. After the resultant water layer was washed with diethyl ether, sodium carbonate was added to the water layer to alkalify it, and the water layer was extracted with methylene chloride. The resultane methylene chloride layer was washed with a saturated aqueous solution of sodium chloride, dried over anhydrous sodium sulfate and then concentrated under reduced pressure to obtain the title compound (4.35 g).
$^1$H-NMR ($CDCl_3$) δ: 1.20-1.50(3H,m), 1.46(9H,s), 1.58-1.69(1H,m), 1.70-1.81(2H,m), 1.98-2.07(1H,m), 2.22-2.31(1H,m), 2.55-2.66(1H,m), 2.97-3.04(1H,m), 3.79-3.93(1H,br), 4.70(1H,d,J=12.0Hz), 4.75-4.85(1H,br), 4.78 (1H,d,J=12.0Hz).
MS (ESI)-M/z: 389[(M,H)$^+$,3xCl$^{35}$], 391[(M+H)$^+$,2xCl$^{35}$,Cl$^{37}$] 393[(M+H)$^+$,Cl$^{35}$,2xCl$^{37}$].

[Referential Example 33]

2,2,2-Trichloroethyl (1S,3R,4S)-3-[(tert-butoxycarbonyl)-aminol-4-({2-[(5-chloropyridin-2-yl)amino]-2-oxoacetyl}-amino) cyclohexanecarboxylate:

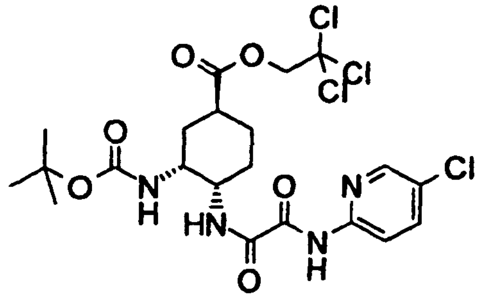

The title compound was obtained by condensing the 32 compound obtained in Referential Example 32 with the compound obtained in Referential Example 28 in a similar manner to the process described in Referential Example 16. $^1$H-NMR ($CDCl_3$) δ: 1.46(9H,s), 1.50-1.63(1H,m), 1.65-1.79(2H,m), 1.87-2.08(2H,m), 2.10-2.22(2H,m), 2.50-2.70 (1H,br), 3.94-4.02(1H,m), 4.17-4.30(1H,br), 4.73(1H,d,J=12.0Hz), 4.78(1H,d,J=12.0Hz), 7.70(1H,dd,J=8.8,2.4Hz), 7.90-8.07(1H,br), 8.18(1H,d,J=8.8Hz), 8.31(1H,d,J=2.4Hz), 9.72(1H,br.s). MS (ESI) m/z: 571[(M+H)$^+$,3xCl$^{35}$], 573[(M+H)$^+$,2xCl$^{35}$,cl$^{37}$], 575[(M+H)$^+$,Cl$^{35}$,2xCl$^{37}$].

Referential Example 34

N-{(1R,2S,5S)-2-[(6-Chloro-2-naphthoyl)amino]-5-[(dimethylamino)carbonyl]cyclohexyl)-5-methyl-4,5,6,7-tetrahydrothiazolo[5,4-c]pyridine-2-carboxamide hydrochloride:

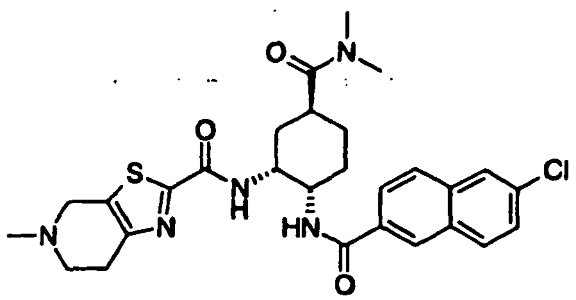

The compound (270 mg) obtained in Referential Example 27 was dissolved in methylene chloride (10 ml), and a 1N ethanol solution (10 ml) of hydrochloric acid was added to stir the mixture for 90 minutes. The solvent was distilled off under reduced pressure, and the resultant residue was "dissolved in N,N-dimethylformamide (7 ml). The compound (110 mg) obtained in Referential Example 10, 1-(3-dimethylaminopropyl)-3-ethylcarbodiimide hydrochloride (100 mg) and 1-hydroxybenzotriazole monohydrate (70 mg) were added to stir the mixture at room temperature for 23 hours. The reaction mixture was concentrated under reduced pressure, and water was added to conduct extraction with ethyl acetate. The resultant organic layer was dried over anhydrous sodium sulfate. The solvent was distilled off under reduced pressure, and the residue was purified twice by column chromatography on silica gel (methylene chloride: methanol = 20:1 → 10:1). The thus-obtained free base was dissolved in methanol, and a 1N ethanol solution (0.30 ml) of hydrochloric acid was added. The residue was washed with ethyl acetate to obtain the title compound (130 mg).
$^1$H-NMR (DMSO-$d_6$) δ: 1.45-1.60(1H,m), 1.70-1.90(3H,m), 1. 90-2.10(2H,m), 2.81(3H,s), 2.91(3H,s), 3.00(3H,s), 3.00-3.22(3H,m), 3.53(2H,br), 4.10-4.20(1H,m), 4.30-4.70(3H,m), 7.59(1H,dd,J=8.8,2.2Hz), 7.87(1H,d,J=8.5Hz), 7.96 (1H,d,J=8.5Hz), 8.02(1H,d,J=8.8Hz), 8.10(1H,d,J=2.2Hz), 8.33(1H,s), 8.43(1H,d,J=8.1Hz), 8.52(1H,d,J=7.3Hz).
MS(FAB)m/z:554(M+H)$^+$.

Referential Example 35

2,2,2-Trichloroethyl (1S,3R,4S)-4-({2-[(5-Chloropyridin-2-yl)amino]-2-oxoacetyl)amino)-3-{[(5-methyl-4,6,6,7-tetrahydrothiazolo[5,4-c]pyridin-2-yl)carbonyl]amino}-cyclohexanecarboxylate:

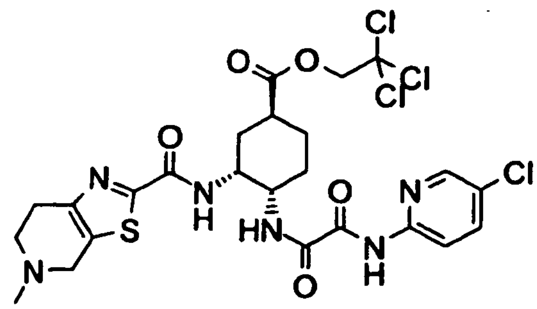

The title compound was obtained by treating the compound obtained in Referential Example 33 with hydrochloric acid to deprotect it, condensing the deprotected compound with the compound obtained in Referential Example 10 and then treating the condensation product with hydrochloric acid again in a similar manner to the process described in Referential Example 34.

$^1$H-NMR ($CDCl_3$) δ: 1.60-1.87(2H,m), 2.04-2.15(2H,m), 2.21-2.32(2H,m), 2.52(3H,s), 2.73-2.89(3H,m), 2.92-2.98(2H,m), 3.71(1H,d,J=15.4Hz), 3.73(1H,d,J=15.4Hz), 4.08-4.16(1H,m), 4.66-4.71(1H,m), 4.72(1H,d,J=12.0Hz), 4.82(1H,d,J=12.0Hz), 7.37(1H,d,J=8.8Hz), 7.69(1H,dd,J=8.8,2.4Hz), 8.05(1H,d,J=8.1Hz), 8.16(1H,d,J-8.8Hz), 8.30(1H,d,J=2.4Hz), 9.69(1H,s).

MS (ESI) m/z: 651[$(M+H)^+$,3x$Cl^{35}$], 653[$(M+H)^+$,2x$Cl^{35}$,$Cl^{37}$], 655[$(M+H)^+$,$Cl^{35}$,2x$Cl^{37}$].

(Example 1]

(1S,3R,4S)-4-({2-[5-Chloropyridin-2-yl]amino}-2-oxoacetyl)amino)-3-{[(5-methyl-4,5,6,7-tetrahydrothiazolo[5,4-c]pyridin-2-yl)carbonyl]amino}-cyclohexane carboxylic acid:

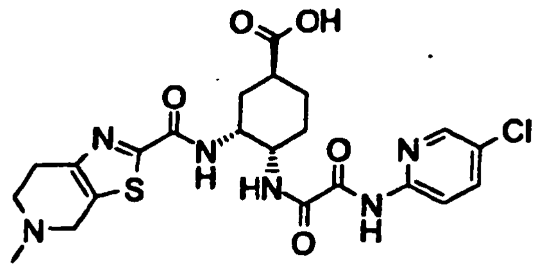

The compound (475 mg) obtained in Referential Example 35 was dissolved in tetrahydrofuran (50 ml), zinc (2.85 g) and acetic acid (5.7 ml) were successively added to the solution, and the mixture was stirred at room temperature for 3 hours. Celite 545 (2.85 g) was added to the reaction mixture to remove insoluble matter by filtration. After the filtrate was concentrated under reduced pressure, methylene chloride was added to the resultant residue, and a 1N aqueous solution of sodium hydroxide was added with stirring to adjust the pH of the reaction mixture to 7. After an organic layer was separated, saturated saline (50 ml) was added to a water layer to conduct extraction with methylene chloride. The resultant organic layers were combined and dried over anhydrous magnesium sulfate. The solvent was distilled off under reduced pressure, and the resultant residue was purified by column chromatography on silica gel (methylene chloride: methanol = 95:5 → 9:1 → 4:1) to obtain the title compound (140 mg).

$^1$H-NMR (DMSO-$d_6$) δ: 1.50-1.80(3H,m), 1.84-1.95(1H,m), 1.95-2.10(1H,m), 2.15-2.30(1H,m), 2.38(3H,s), 2.40-2.50 (1H,m), 2.67-2.80(2H,m), 2.80-2.95(2H,m), 3.66(2H,m), 4.03(1H,br.s), 4.33(1H,br.s), 7.97-8.10(2H,m), 8.45(1H,s), 8.53(1H,d,J=6.8Hz), 9.19(1H,d,J=8.3Hz), 10.27(1H,br.s).
MS (FAB) m/z: 521[(M+H)$^+$,$^{35}$Cl], 523[(M+H)$^+$,$^{37}$Cl].

[Test Example 1]

Determination of human FXa-inhibiting effect ($IC_{50}$ value):

5% DMSO solutions (10 µl) of each test compound, the concentrations of which were suitably set stepwise, Tris buffer (100 mM Tris, 200 mM potassium chloride, 0.2% BSA, pH 7.4) (40 µl) and 0.0625 U/ml human FXa (Enzyme Research Labolatories, Inc., dissolved and diluted with Tris buffer) (10 µl) were respectively put in wells of a 96-well microplate, and a 750 µM aqueous solution (40 µl) of S-2222 (Chromogenix Co.) was added. Absorbance at 405 nm was measured for 10 minutes at room temperature to determine an increase in absorbance (ΔOD/min). As a control, Tris buffer was used in place of the test compound.

The percent inhibition (%) calculated using the following equation at the final concentration of the test compound and the final concentration of the test compound were plotted on the axis of ordinate and the axis of abscissa of logarithmic normal probability paper, respectively, to determine the 50 % inhibition dose ($IC_{50}$ value).

Percent inhibition (%) =

[1 - (ΔOD/min of test compound) ÷ (ΔOD/min of control)]

x 100

(Result)

It may be demonstrated that the compounds disclosed herein have a potent FXa-inhibiting effect.

[Test Example 2]

Determination of anti-FXa activity in rat plasma after oral administration:

(A) Administration and blood collection:

A drug solution (1 mg/ml) obtained by dissolving or suspending a test compound (10 mg) in 0.5% methyl cellulose (MC) was orally administered to rats (10 ml/kg). After 0.5, 1, 2 and 4 hours from the drug administration, the blood (0.5 ml) was collected through the jugular vein using a syringe which is containing a 3.13% (w/v) aqueous solution (50 µl) of trisodium citrate dihydrate (amount of blood collected: 0.45 ml). For rats of a control group, the same blood collection was conducted after a 0.5% MC solution was administered. Each blood sample was centrifuged at 1500 x g for 10 minutes at 4°C to separate plasma, and the plasma was preserved at -40°C until it was used in the following determination of anti-FXa activity in plasma.

(B) Determination of FXa-inhibiting activity in plasma:

In the determination of anti-FXa activity in plasma, S-2222 was used as a substrate. Tris buffer (100 mM Tris, 200 mM potassium chloride, 0.2% BSA, pH 7.4) (5456 µl), human FXa (2.5 U/ml, 44 µl) and water (550 µl) were mixed. The resultant human FXa solution was used in the following test.

Rat plasma (5 µl) obtained in accordance with the procedure (A) described above was put in wells of a 96-well microplate, and the above-described human FXa solution (55 µl) and a 750 µM aqueous solution (40 µl) of S-2222 were sequentially added. Immediately after that, absorbance at 405 nm was measured at room temperature by means of a spectrophotometer SPECTRAmax 340 or 190 (Molecular Devices Co., U.S.A.), thereby determining a rate of reaction (ΔOD/min).

The anti-FXa activity, i.e., percent inhibition (%) was calculated in accordance with the following equation:

$$\text{Percent inhibition (\%)} = [1 - (\Delta OD/\text{min of sample}) \div (\text{average value of } \Delta OD/\text{min of the control group})] \times 100$$

INDUSTRIAL APPLICABILITY

The cyclicdiamine derivatives described herein exhibit a potent inhibitory effect on activated blood coagulation factor X and are useful as medicines, activated blood coagulation factor X inhibitors, anticoagulants, agents for preventing and/or treating thrombosis or embolism, agents for preventing and/or treating thrombotic disease and agents for preventing and/or treating cerebral infarction, cerebral embolism, myocardial infarction, angina pectoris, pulmonary infarction, pulmonary embolism, Buerger's disease, deep venous thrombosis, disseminated intravascular coagulation syndrome, thrombus formation after valve or joint replacement, thrombus formation and reocclusion after angioplasty, systemic inflammatory response syndrome (SIRS), multiple organ dysfunction syndrome (MODS), thrombus formation during extracorporeal circulation, or blood clotting upon blood drawing.

**Claims**

1. A compound having a stereochemical structural formula, including a salt thereof, which is:

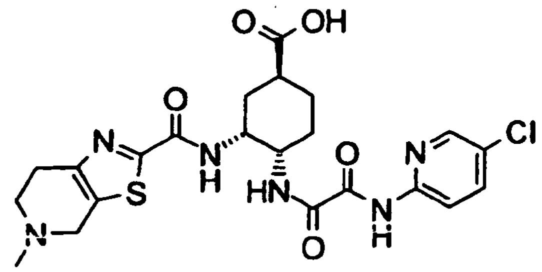

2. A composition comprising at least-a compound according to claim 1 and a pharmaceutica!)y acceptable carrier.

3. The composition according to claim 2, wherein said composition further comprises at least one pharmaceutically acceptable additive selected from the group consisting of fillers, extenders, binders, disintegrating agents, dissolution aids/accelerators, suspending agents, emulsifying agents, wetting agents, stabilizers, and preservatives.

4. The composition according to claim 2, wherein said composition is in the form of a tablet, a granule, a capsule, a powder, a solution, a suspension, an emulsion, an oil, a syrup, an elixir, an ointment, a gel, a cream, a lotion, a spray, or a plaster.

5. The composition according to claim 2, wherein said composition is suitable for oral, topical, or injection administration.

6. The composition according to any one of claims 2 to 5 for use in the treatment of thrombosis or embolism by administering to a mammal in need thereof an effective amount of a compound according to claim 1.

7. The composition according to claim 6, wherein said effective amount ranges from 1 mg to 1000 mg per day of said at least one compound present within said composition.

8. The composition according to claim 6, wherein said effective amount ranges from 0.1 mg to 200 mg per kg of body weight of said mammal per day of said at least one compound present within said composition.

9. The composition according to claim 6, wherein the administration of said composition ranges from one to four times

per day.

**10.** A process for preparing a composition, comprising combining at least one compound according to claim 1 with a pharmaceutically acceptable carrier.

**11.** The process according to claim 10, wherein said process further comprises combining with said at least one compound and said pharmaceutically acceptable carrier, at least one pharmaceutically acceptable additive selected from the group consisting of fillers, extenders, binders, disintegrating agents, dissolution aids/accelerators, suspending agents, emulsifying agents, wetting agents, stabilizers, and preservatives.

**Patentansprüche**

**1.** Verbindung mit folgender stereochemischen Strukturformel, einschließlich ein Salz davon:

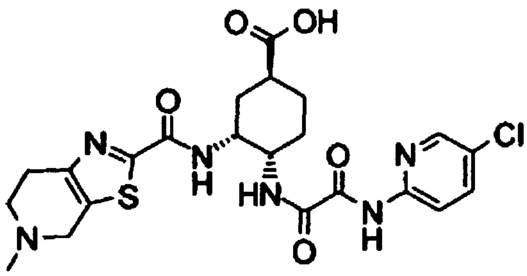

**2.** Zusammensetzung, umfassend mindestens eine Verbindung nach Anspruch 1 und einen pharmazeutisch unbedenklichen Träger.

**3.** Die Zusammensetzung nach Anspruch 2, wobei die Zusammensetzung ferner mindestens ein pharmazeutisch unbedenkliches Additiv umfasst, das ausgewählt ist aus der Gruppe, bestehend aus Füllstoffen, Streckmitteln, Bindemitteln, Sprengmitteln, Lösungshilfen/Beschleunigern, Suspensionsmitteln, Emulgatoren, Benetzungsmitteln, Stabilisatoren und Konservierungsstoffen.

**4.** Die Zusammensetzung nach Anspruch 2, wobei die Zusammensetzung in Form einer Tablette, einer Granule, einer Kapsel, eines Pulvers, einer Lösung, einer Suspension, einer Emulsion, eines Öls, eines Sirups, eines Elexiers, einer Salbe, eines Gels, einer Creme, einer Lotion, eines Sprays oder eines Pflaster vorliegt.

**5.** Die Zusammensetzung nach Anspruch 2, wobei die Zusammensetzung zur oralen, topischen oder Verabreichung durch die Injektion geeignet ist.

**6.** Die Zusammensetzung nach einem der Ansprüche 2 bis 5 zur Verwendung bei der Behandlung von Thrombose oder Embolie durch Verabreichung an ein Säugetier, das dieser Bedarf, einer wirksamen Menge der Verbindung nach Anspruch 1.

**7.** Die Zusammensetzung nach Anspruch 6, wobei die wirksame Menge 1 mg bis 1000 mg pro Tag der mindestens einen Verbindung, die in der Zusammensetzung vorliegt, beträgt.

**8.** Die Zusammensetzung nach Anspruch 6, wobei die wirksame Menge 0,1 mg bis 200 mg pro Kg Körpergewicht des Säugetiers pro Tag der mindestens einen Verbindung, die in der Zusammensetzung vorliegt, beträgt.

**9.** Die Zusammensetzung nach Anspruch 6, wobei die Verabreichung der Zusammensetzung einmal bis viermal pro Tag erfolgt.

**10.** Verfahren zur Herstellung einer Zusammensetzung, umfassend eine Kombination mindestens einer Verbindung nach Anspruch 1 mit einem pharmazeutisch unbedenklichen Träger.

11. Das Verfahren nach Anspruch 10, wobei das Verfahren ferner eine Kombination mit der mindestens einen Verbindung und dem pharmazeutisch unbedenklichen Träger von mindestens einem pharmazeutisch unbedenklichen Additiv umfasst, das ausgewählt ist aus der Gruppe, bestehend aus Füllstoffen, Streckmitteln, Bindemitteln, Sprengmitteln, Lösungshilfen/Beschleunigern, Suspensionsmitteln, Emulgatoren, Benetzungsmitteln, Stabilisatoren und Konservierungsstoffen.

**Revendications**

1. Composé, y compris un sel de celui-ci, ayant une formule structurelle stéréochimique qui est la suivants :

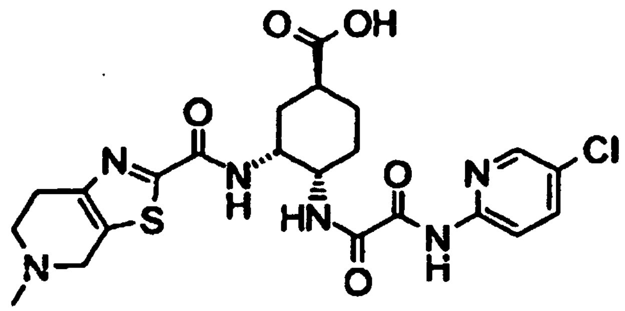

2. Composition comprenant au moins un composé selon la revendication 1 et un véhicule pharmaceutiquement acceptable.

3. Composition selon la revendication 2, laquelle composition comprend en outre au moins un additif pharmaceutiquement acceptable choisi dans l'ensemble constitué par les charges, les agents de remplissage, les liants, les agents délitants, les auxiliaires/ accélérateurs de dissolution, les agents de mise en suspension, les agents émulsionnants, les agents mouillants, les stabilisants, et les conservateurs.

4. Composition selon la revendication 2, laquelle composition est sous la forme d'un comprimé, d'un granule, d'une capsule, d'une poudre, d'une solution, d'une suspension, d'une émulsion, d'une huile, d'un sirop, d'un élixir, d'une pommade, d'un gel, d'une crème, d'une lotion, d'un spray, ou d'un emplâtre.

5. Composition selon la revendication 2, laquelle composition est adaptée pour une administration par voie orale, par voie topique ou par injection.

6. Composition selon l'une quelconque des revendications 2 à 5, pour utilisation dans le traitement d'une thrombose ou d'une embolie par administration, à un mammifère en ayant besoin, d'une quantité efficace d'un composé selon la revendication 1.

7. Composition selon la revendication 6, dans laquelle ladite quantité efficace est située dans la plage allant de 1 mg à 1000 mg par jour dudit au moins un composé présent dans ladite composition.

8. Composition selon la revendication 6, dans laquelle ladite quantité efficace est située dans la plage allant de 0,1 mg à 200 mg, par kg de poids corporel dudit mammifère et par jour, dudit au moins un composé présent dans ladite composition.

9. Composition selon la revendication 6, dans laquelle l'administration de ladite composition est effectuée une à quatre fois par jour.

10. Procédé pour préparer une composition, comprenant la combinaison d'au moins un composé selon la revendication 1 avec un véhicule pharmaceutiquement acceptable.

11. Procédé selon la revendication 10, lequel procédé comprend en outre la combinaison, avec ledit au moins un composé et ledit véhicule pharmaceutiquement acceptable, d'au moins un additif pharmaceutiquement acceptable choisi dans l'ensemble constitué par les charges, les agents de remplissage, les liants, les agents délitants, les

auxiliaires/accélérateurs de dissolution, les agents de mise en suspension, les agents émulsionnants, les agents mouillants, les stabilisants, et les conservateurs.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0009480 A
- WO 9830574 A


### Non-patent literature cited in the description


- *Thrombosis Research,* 1992, vol. 68, 507-512
- *Thrombosis Research,* 1979, vol. 15, 617-629
- *Journal of Clinical Investigation,* 1983, vol. 71, 1383-1389
- *Mebio,* vol. 14, 92-97
- *Science,* 1990, vol. 248, 593-596
- *Journal of Biological Chemistry,* 1988, vol. 263, 10162-10167
- *Organic Synthesis,* 1995, vol. III, 217
- *J. Org. Chem.,* 1998, vol. 63, 6094
- *Tetrahedron: Asymmetry,* 1996, vol. 7, 843
- *J. Org. Chem.,* 1985, vol. 50, 4154
- *J. Med. Chem.,* 1998, vol. 41, 38
- *J. Org. Chem.,* 1996, vol. 61, 8687-8691
- *J. Org. Chem.,* 1996, vol. 61, 8687
- *Eur. J. Chem-Chim. Ther.,* 1984, vol. 19, 205-214